Europäisches Patentamt

European Patent Office

Office européen des brevets

(19)

(11) Publication number: **0 255 694**
**A1**

# EUROPEAN PATENT APPLICATION

(21) Application number: 87110994.8

(22) Date of filing: 29.07.87

(51) Int. Cl.4: **C12N 15/00** , A61K 39/395 ,
C12N 5/00 , C12P 21/00

(30) Priority: 30.07.86 JP 177809/86
31.07.86 JP 178881/86
01.08.86 JP 180194/86

(43) Date of publication of application:
10.02.88 Bulletin 88/06

(84) Designated Contracting States:
BE CH DE FR GB LI SE

(71) Applicant: TEIJIN LIMITED
11 Minami Honmachi 1-chome Higashi-ku
Osaka-shi Osaka-fu(JP)

(72) Inventor: Nishimura, Yushi
First Mansion 103 399-1, Oaza Miya
Hino-shi Tokyo(JP)
Inventor: Ichikawa, Yataro
2-11-7, Kotesashi-cho
Tokorozawa-shi Saitama(JP)
Inventor: Kudo, Akira
Claragraden 114
CH-4057 Basel(CH)
Inventor: Watanabe, Takeshi
870-46, Yaemizo Nabeshima-cho
Saga-shi Saga(JP)

(74) Representative: Vossius & Partner
Siebertstrasse 4 P.O. Box 86 07 67
D-8000 München 86(DE)

(54) Mouse-human chimera antibody and its components and gene therefor.

(57) A mouse-human chimera antibody reactive with a human common acute lymphocytic leukemia antigen, comprising a H chain comprising a V region of mouse origin and a C region of human origin; and an L chain comprising a V region of mouse origin and a C region of human origin; and components thereof; and a gene coding for their components, as well as a process for production of the chimera antibody.

# MOUSE-HUMAN CHIMERA ANTIBODY AND ITS COMPONENTS AND GENE THEREFOR

The present invention relates to mouse-human chimera antibody and its components, as well as DNA fragments coding for and capable of expression of these components, plasmids containing these DNA fragments, and cells transformed with these plasmids.

Immunoglobulin comprises two H chains and two L chains, and both chains comprise a variable region (V region) and a constant region (C region).

In the study of genes of mouse antibodies, various kinds of gene units including those contained in the V region gene and C region gene, as well as a promoter, enhancer, etc., have been found, and nucleotide sequences of some of them have been already determined.

Recently, monoclonal antibody-producing technology has progressed, and the application of monoclonal antibodies has become promising for the immunological diagnosis, control and prophylaxis of various diseases. For these applications, human monoclonal antibodies are most preferable. The technology for the production of human monoclonal antibodies, however, has not progressed as far as that of mouse monoclonal antibodies. Therefore, only a limited number of antibodies having different specificities can be obtained, resulting in a limited application of human monoclonal antibodies for immunological diagnosis, treatment, prophylaxis, etc. of diseases. In an attempt to overcome these difficulties, consideration has been given to the artificial construction of antibodies similar to human antibodies and, therefore, not recognized as exogenous material, and the use of such artificial antibodies for immunological diagnosis, treatment, and the like. As one such antibody, JP-A-60-155132 discloses a chimera antibody comprising V regions of a mouse antibody and C regions of a human antibody. Said reference discloses a chimera peptide comprising V regions of mouse origin of an antibody reactive with melanoma cells and C regions of human origin. However, the biological activity of this peptide has not been confirmed.

Several of the present inventors described a mouse-human chimera antibody H chain comprising an amino acid sequence of a V region of an H chain specifically reactive with a human common acute lymphocyctic leukemia antigen (cALLA), and a C region of an H chain of a human antibody, in Japanese Patent Application No. 61-41983 filed on February 28, 1986.

The present invention relates to a mouse-human chimera antibody reactive with a human common acute lymphocytic leukemia antigen, comprising two H chains comprising a V region of mouse origin and a C region of human origin; and two L chains comprising a V region of mouse origin and a C region of human origin.

The present invention also provides a chimera mouse-human antibody L chain comprising a V region of a mouse antibody reactive with a human common acute lymphocytic leukemia antigen, and a C region of human origin.

The present invention further provides a V region of an L chain of a mouse antibody reactive with a human common acute lymphocytic leukemia antigen.

Furthermore, the present invention provides a chimera mouse-human antibody H chain comprising a V region of a mouse antibody reactive with a human common acute lymphocytic leukemia antigen and a C region of human origin.

The present invention also provides a V region of an H chain of a mouse antibody reactive with a human common acute lymphocytic leukemia antigen.

The present invention further provides a nucleotide sequence for expression of a chimera mouse-human L chain wherein a V region of the L chain is that of a mouse antibody reactive with a human common acute lymphocytic leukemia antigen, comprising

a DNA fragment coding for a V region of the L chain of mouse origin;

a DNA fragment coding for a C region of the L chain of human origins; and

an enhancer DNA sequence of human H chain gene origin.

The present invention provides a nucleotide sequence for expression of a chimera mouse-human H chain wherein a V region of the H chain is that of a mouse antibody reactive with a human common acute lymphocytic leukemia antigen, comprising

a DNA fragment coding for a V region of the H chain of mouse origin;

a DNA fragment coding for a C region of the H chain of human origin; and

an enhancer DNA sequence of human H chain gene origin; and optically,

an enhancer DNA sequence of mouse H chain gene origin.

The present invention also provides a nucleotide sequence coding for a V region of an L chain of a mouse antibody reactive with a human common acute lymphocytic leukemia antigen.

The present invention further provides plasmids containing one of the above-mentioned nucleotide sequences.

The present invention also provides animal cells transformed with the above-mentioned plasmids capable of producing a chimera H chain or chimera antibody reactive with a human common acute lymphocytic leukemia antigen.

Further, the present invention provides a process for the production of the chimera antibody using the animal cells.

Figure 1 represents an amino acid sequence of a V region of an H chain ($\gamma_{2a}$ chain) of a mouse antibody;

Fig. 2 represents an amino acid sequence of a C region of an H chain ($\gamma_1$ chain) of a human antibody;

Fig. 3 represents an amino acid sequence of a V region of an L chain ($x$ chain) of a mouse antibody;

Fig. 4 represents an amino acid sequence of a C region of an L chain ($x$ chain) of a human antibody;

Fig. 5 represents an enhancer nucleotide sequence of an H chain of a human antibody;

Fig. 6 represents a nucleotide sequence of a V region gene of a H chain of a mouse antibody;

Fig. 7 represents a nucleotide sequence of a C region gene of an H chain of a human antibody;

Fig. 8 represents a nucleotide sequence of a V region gene of an L chain of a mouse antibody;

Fig. 9 represents a nucleotide sequence of a C region of an L chain of a mouse antibody;

Fig. 10 represents a restriction enzyme cleavage map of a V region gene of an L chain of a mouse antibody;

Fig. 11 represents a restriction enzyme cleavage map of a C region gene of an L chain of human antibody;

Fig. 12 represents a restriction enzyme cleavage map of a gene containing a V region gene of an H chain of a mouse antibody;

Fig. 13 represents the construction of an L chain gene of a chimera antibody;

Fig. 14 represents the construction of an H chain gene of a chimera antibody;

Fig. 15 represents the result of a Northern blot analysis of chimera antibody H chain gene transcription products;

Fig. 16 represents the result of SDS-polyacrylamide gel electrophoresis for translation products of a chimera antibody H chain gene;

Fig. 17 represents the result of a Northern blot analysis of chimera antibody L chain transcription products;

Fig. 18 represents the result of SDS-polyacrylamide gel electrophoresis for a chimera antibody (H + L chains) produced by X63Ag8.653-HL cells;

Fig. 19 represents the result of flow cytometry analysis for the ability of chimera antibody to bind to target cells; and

Fig. 20 is a graph showing the result of a therapeutical experiment with the present antibody using experimental animals.

In the present specification, when amino acids and peptides are expressed by an abbreviation, such abbreviations are used according to the recommendations of the IUPAC-IUB (Commission on Biological Nonmenclature). For example, the following abbreviations are used.

Ala L-alanine
Arg L-arginine
Asn L-asparagine
Asp L-aspartic acid
Cys L-cysteine
Gln L-glutamine
Glu L-glutamic acid
Gly glycine
His L-histidine
Ile L-isoleucine
Leu L-leucine
Lys L-lysine
Met L-methionine
Phe L-phenylalanine
Pro L-proline
Ser-L-serine
Thr L-threonine
Trp L-tryptophan
Tyr L-tyrosine
Val L-valine.

Further, the nucleotide sequence is shown by abbreviations representing the kinds of bases contained in deoxyribonucleotide. For example, the following abbreviations are used:

A adenine (deoxyadenylic acid)
C cytosine (deoxycytidylic acid)
G guanine (deoxyguanylic acid)
T thymine (deoxythymidylic acid).

I. Chimera antibody

The present invention relates to a mouse-human chimera antibody reactive with a human common acute lymphocytic leukemia antigen, comprising two H chains comprising a V region of mouse origin and a C region of human origin; and two L chains comprising a V region of mouse origin and a C region of human origin.

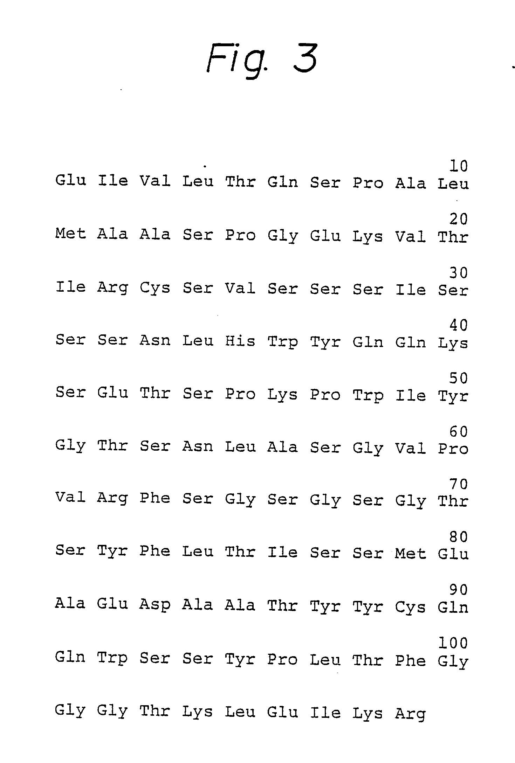

The V region of the L chain is accompanied by a J region, to form a V-J region. For example, the L chain V region is of mouse origin and the amino acid sequence thereof comprises an amino acid sequence from the first amino acid (Glu) to the 97th amino acid (Leu) shown in Fig. 3. The V-J region amino acid sequence consists of an amino acid sequence from a first amino acid (Glu) to the 109th amino acid (Arg) shown in Fig. 3. Therefore the amino acid sequence from the 98th acid (Thr) to the 109th acid (Arg) represent a J amino acid sequence.

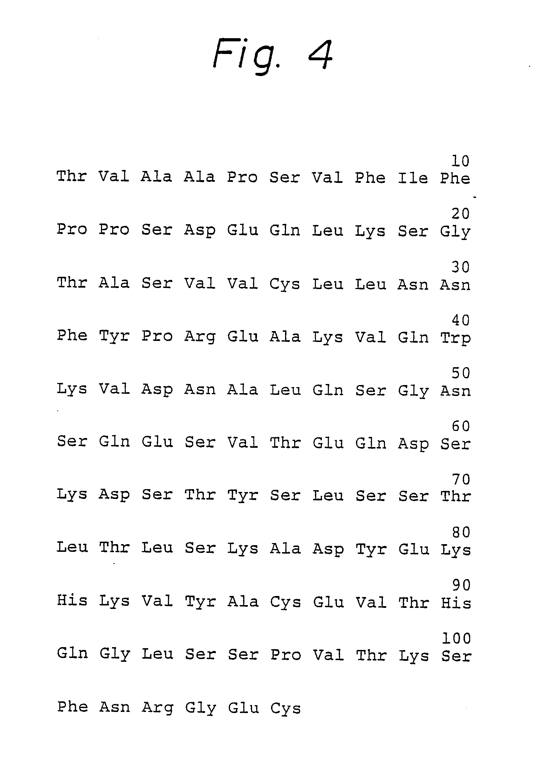

The C region of the L chain is derived from, for example, a x chain of human origin, and contains, for example, the amino acid sequence represented in Fig. 4.

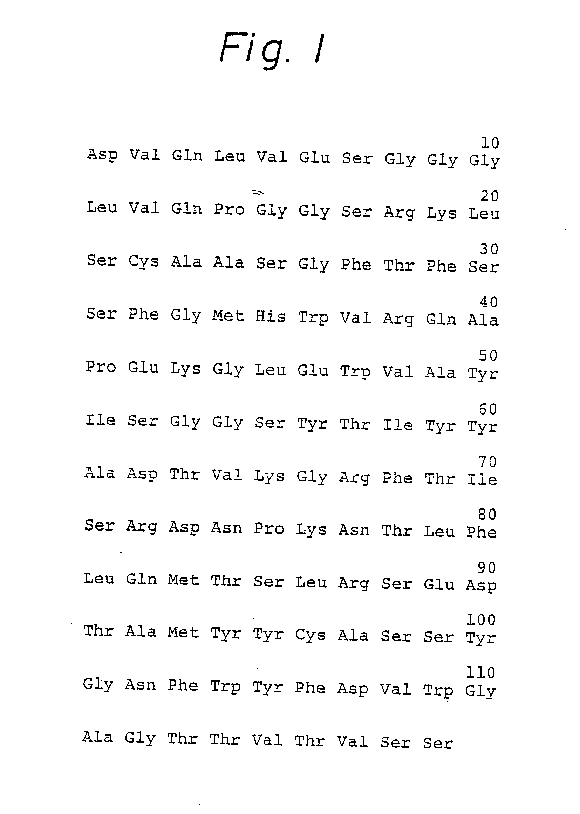

The V region of the H chain is that of a mouse antibody reactive with a human common acute lymphocyte leukemia antigen, and has, for example, the amino acid sequence shown in Fig. 1.

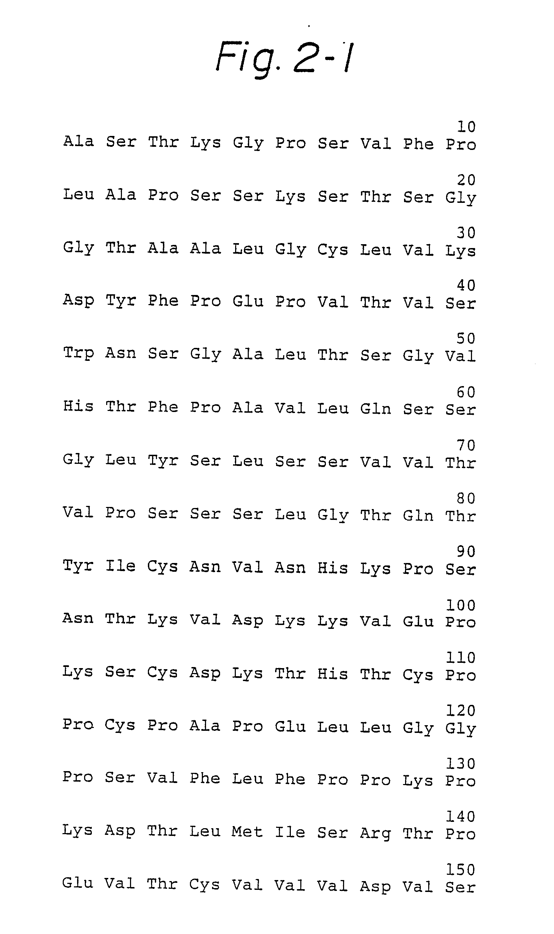

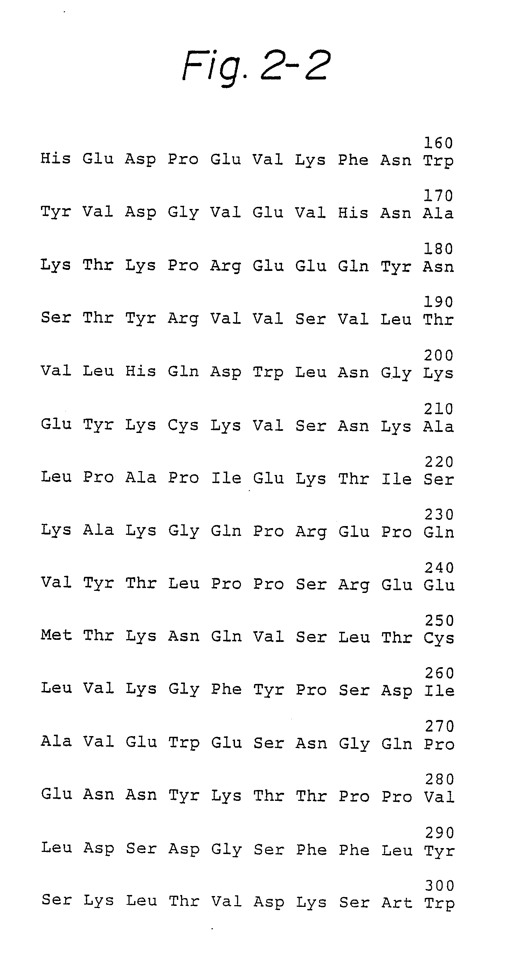

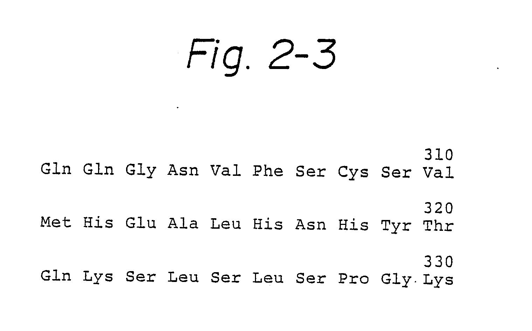

The C region of the H chain is derived from, for example, a C $_{\gamma 1}$ chain of a human antibody. An amino acid sequence of the C region of the H chain is exemplified in Fig. 2.

The present invention also relates to nucleotide sequences coding for the above-mentioned mouse-human chimera antibody. According to an embodiment of the present invention, a plasmid containing a gene coding for the L chain and a plasmid containing a gene coding for the H chain are separately constructed, and both plasmids are introduced to the same animal cells to transform animal cells capable of producing the target chimera antibody. Therefore, in this specification, the nucleotide sequence coding for the L chain and the nucleotide sequence coding for the H chain are separately described.

## II. Nucleotide sequence coding for mouse-human chimera antibody H chain

A nucleotide sequence for expression of a mouse-human chimera H chain, wherein the V region of the H chain is that of a mouse antibody reactive with a human common acute lymphocytic leukemia antigen, comprises
a DNA fragment coding for the V region of the H chain of mouse origin;
a DNA fragment coding for the C region of the H chain of human origin; and
an enhancer DNA sequence of the human H chain gene origin; and optionally,
an enhancer DNA sequence of mouse H chain gene origin.

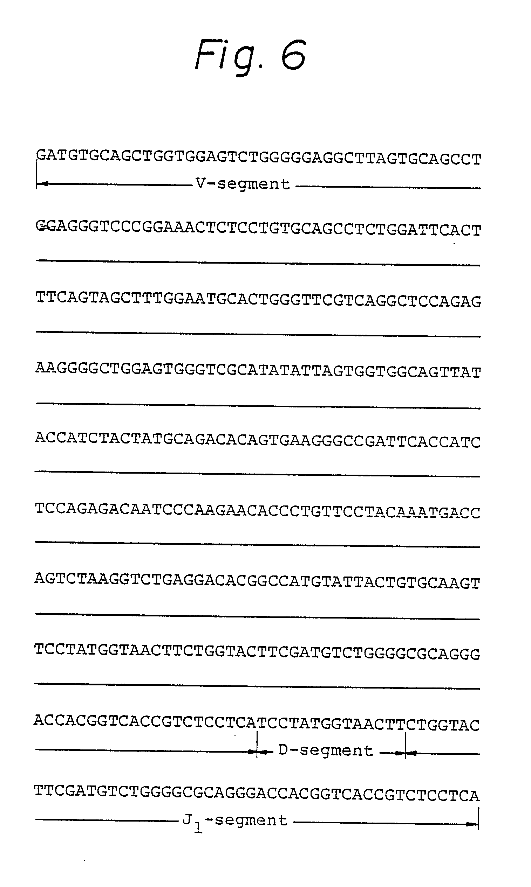

The DNA fragment coding for the V region of the H chain is exemplified by the nucleotide sequence shown in Fig. 6, and a complementary sequence thereof. As shown in Fig. 6, this nucleotide sequence comprises a V-segment, D-segment, and J$_1$-segment.

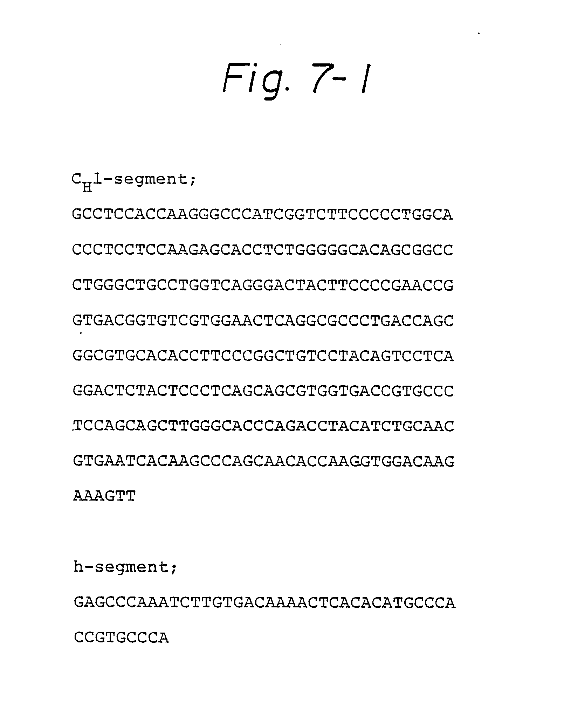

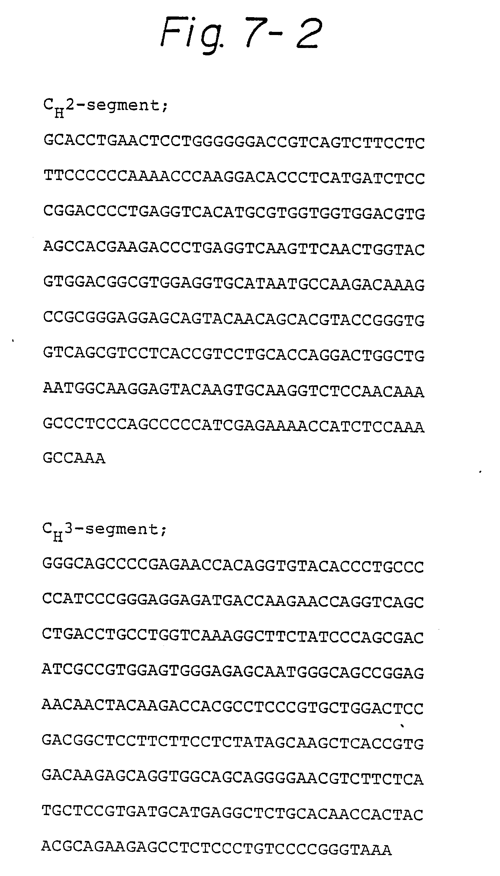

The DNA fragment coding for the C region of the H chain is exemplified by the nucleotide sequence shown in Fig. 7-1 and Fig. 7-2, and a complementary sequence thereof. As shown in Figs. 7-1 and 7-2, this nucleotide sequence comprises a C$_H$1-segment, hinge(h)-segment, C$_H$2-segment, and C$_H$3-segment.

The nucleotide sequence coding for the H chain comprises, in addition to the above-mentioned DNA fragment coding for the V region and DNA fragment coding for the C region, an enhancer DNA sequence of human antibody H chain gene origin, and optionally, an enhancer DNA sequence of mouse antibody H chain gene origin, to enhance the expression efficiency. Although the enhancer(s) can be positioned at any site(s) relating to the above-mentioned coding DNA fragments, in a preferable embodiment, they are positioned between the DNA fragment coding for the V region and the DNA fragment coding for the C region.

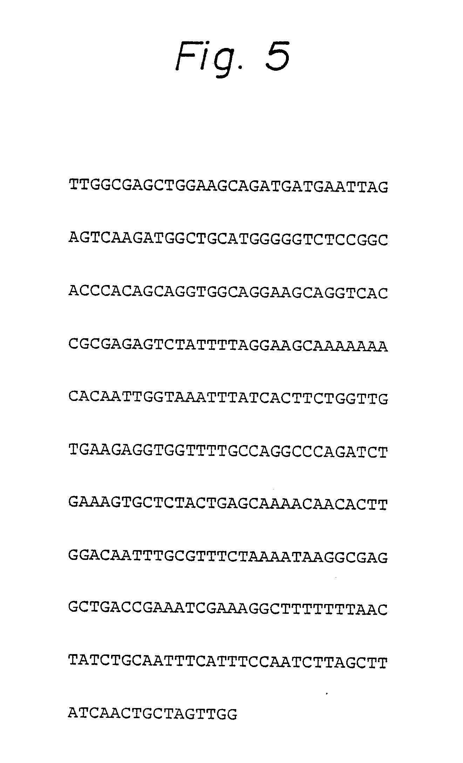

The enhancer DNA sequence is exemplified by the nucleotide sequence shown in Fig. 5, and a complementary sequence thereof.

The nucleotide sequence coding for the H chain con contain, in addition to the above mentioned components, introns of human and mouse gene origin.

## III. Nucleotide sequence coding for mouse-human chimera antibody L chain

A nucleotide sequence for expression of a mouse-human chimera L chain wherein the V region of the L chain is that of a mouse antibody reactive with a human common acute lymphocytic leukemia antigen, comprises,
a DNA fragment coding for the V region of the L chain of mouse origin;
a DNA fragment coding for the C region of the L chain of human origin; and
an enhancer DNA sequence of human H chain gene origin.

The DNA fragment coding for the V region of the L chain is usually accompanied by a J region. The V region contains the amino acid sequence from the first amino acid (Glu) to the 97th amino acid (Leu) shown in Fig. 3. The V-J region contains the amino acid sequence from the first amino acid (Glu) to the 109th amino acid (Arg) shown in Fig. 3. The J region has the amino acid sequence from the98th amino acid (Thr) to the 109th amino acid (Arg) shown in Fig. 3.

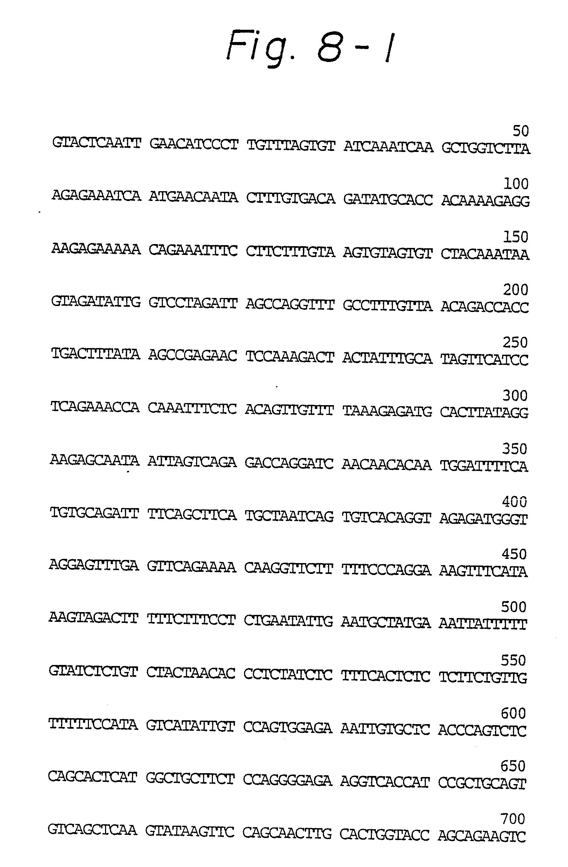

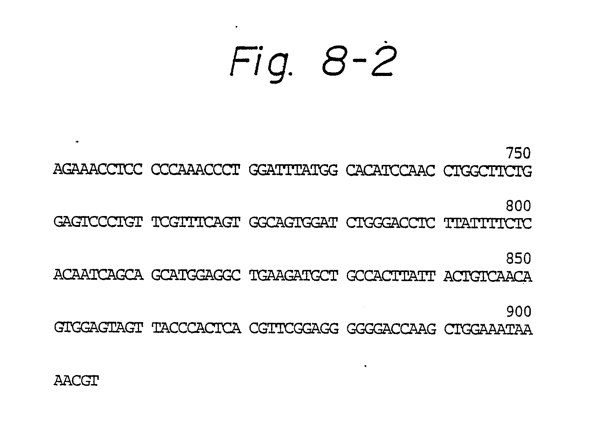

The DNA fragment coding for the V region comprises, for example, the nucleotide sequence from the 579th nucleotide (G) to the 869th nucleotide (C) shown in Fig. 8A. The DNA fragment coding for the V-J region amino acid sequence comprises the nucleotide sequence from the 579th nucleotide (G)to the 905th nucleotide (T) shown in Fig. 8A. The nucleotide sequence from the 870th nucleotide to the 905th nucleotide (T) represents the DNA portion coding for the J region amino acid sequence.

Moreover, the DNA fragment coding for the V-J region amino acid sequence can comprise the nucleotide sequence from the 562th nucleotide (T) to the 905th nucleotide (T), the nucleotide sequence from the 340th nucleotide (A) to the 905th nucleotide (T), or the nucleotide sequence from the 230th nucleotide (T) to the 905th nucleotide (T).

The above-mentioned DNA fragments can be obtained from cells of a mouse hybridoma cells line such as NL-1 line, according to the procedure as described in Examples 1 to 6.

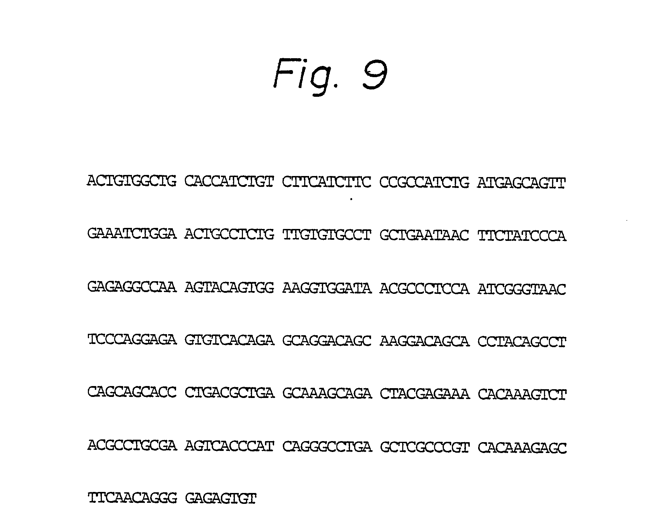

The DNA fragment coding for the C region of the L chain is preferably of human antibody x chain gene origin, and, for example, codes for an amino acid sequence shown in Fig. 4. The DNA fragment is exemplified by, for example, a nucleotide sequence shown in Fig. 9.

The present DNA sequence capable of expressing the L chain contains an enhancer DNA sequence derived from an H chain gene of human or mouse origin. This enhancer DNA sequence may be any DNA sequence present in the H chain gene of human or mouse origin and having an enhancer function; for example, the enhancer DNA sequence derived from the H chain gene of human origin disclosed in EP-A-146743, preferably the DNA sequence consisting of the DNA sequence of the H chain gene of human origin and the complementary sequence thereof as shown in Fig. 5. The enhancer DNA sequence can be positioned at any site in the present DNA sequence for expression of the L chain; i.e., it can be positioned between the V region-coding DNA fragment and the C region-coding DNA fragment, or it can be positioned either at the 5′ to the V region-coding DNA fragment or at the 3′ to the C region-coding DNA fragment in the DNA sequence comprising the V region-coding DNA sequence present at the upstream position and the C region-coding DNA sequence present at the downstream position.

Moreover, the present DNA sequence for expression of the L chain can contain DNA fragments having a function other than the above-mentioned functions, for example, a promoter, another enhancer, intron, and the like.

The present DNA sequence for expression of the L chain is inserted to an appropriate plasmid such as pSV2gpt, pSV2neo, pKSV-10, or the like to construct a recombinant plasmid capable of expressing the L chain when introduced into appropriate host cells. The construction processes of such plasmids are described in detail in Examples 1 to 5 and 7 to 13.

The thus-constructed plasmid is introduced into host animal cells such as mouse myeloma cells, for example, the J558L, NS-1, or X63Ag8.653 cell line, according to a conventional procedure such as the protoplast method (see, MEN-EKI JIKKEN SOSA HO XIII p 4533, Japanese Society for Immunologist, 1984) or the DEAE-dextran method (see, Cell, Vol 33, p 729, 1983) to obtain transformed cells as described in detail in Example 25.

The transformant cells thus prepared are cultured in an appropriate conventional medium to accumulate the target antibody, which is then recovered an purified by a conventional process.

The mouse-human chimera antibody of the present invention not only can be used for diagnosis and therapy of human common acute lymphocytic leukemia, but also is promising, by conjugating it to an anticancer agent, for application in immunological therapy for human common acute lymphocytic leukemia.

Examples

The present invention will now be further illustrated by, but is by no means limited to, the following examples.

Example 1 Isolation of mouse chromosomal DNA

1 x 10[8] cells of a mouse hybridoma cell line NL-1 were treated with protease K (Sigma) in the presence of 1% sodium lauryl sulfate (SDS), and to the mixture was added phenol saturated with water to extract proteins. The whole was then centrifuged to separate the aqueous phase, which was then dialyzed in a buffer containing 10 mM Tris-HCl (pH 7.2), 0.1 mM NaCl and 0.1 mM EDTA (TNE buffer). The dialyzate was treated with ribonuclease A (Sigma), and after extraction with phenol, dialyzed in a TNE buffer to obtain 1.2 mg of mouse chromosomal DNA (see, N. Blin, D.W. Stafford, Nucleic Acids Res. Vol 3, 2303, 1976).

## Example 2   Construction of mouse gene library

150 μg of the mouse chromosomal DNA obtained in Example 1 was digested with a restriction enzyme HindIII (Takara Shuzo, Japan) according to the same procedure as later described in Example 4, and the digestion product was subjected to sucrose density-gradient centrifugation (sucrose concentration 10 - 40%, wt/vol; 28000 rpm, 120000 Xg; 15 hours; 20°C) to obtain DNA fragments corresponding to 4 to 9 Kb.

Next, 0.45 μg of the DNA fragment thus obtained were ligated with a Hind III arm of the Charom 28 vector (Bethesda Research Laboratories), and the reaction product was subjected to in vitro packaging using an Amersham kit to obtain 4 x 10⁶ PFU/μg of a mouse NL-1 gene library. The above-mentioned ligation was carried out in a reaction mixture containing 66 mM Tris-HCl (pH 7.6), 6.6 mM MgCl₂ , 10 mM dithiothreitol, and 1 mM ATP, at 4°C for 16 hours.

## Example 3   Screening of gene for mouse immunoglobulin x chain

A Charon 28 phage containing DNA derived from the mouse cell line NL-1 prepared in Example 2, was infected to Echerichia coli LE392, and positive clones were selected using a $^{32}$P-labeled x chain J gene of a human antibody labeled with $^{32}$P by nick-translation (Amersham) according to a plaque hybridization method (see, W.D. Benton, R.W. Davis, Science, Vol 196, 180, 1977). From the selected Charon 28 phage, a phage DNA containing a gene for mouse immunoglobulin x chain was isolated according to the Thomas and Davis method (M. Thomas and R.W. Davis, J. Mol. Biol., 91, 315, 1974).

## Example 4   Preparation of restriction enzyme cleavage map of mouse x chain V region gene

The phage DNA containing the mouse x chain gene obtained in Example 3, as well as the DNA prepared by recloning the mouse x chain gene to a plasmid according to the same procedure as described in Example 5 (both cases, 1 μg) were separately dissolved in 20 μl of a restriction enzyme cleavage buffer, and digested with 2 units of a restriction enzyme at 37°C for at least one hour.

The restriction enzyme cleavage buffer was an aqueous buffer solution containing 50 mM Tris-HCl (pH 7.4), 100 mM NaCl, and 10 mM MgSO₄ for XbaI and BglII; or an aqueous buffer solution containing 10 mM Tris-HCl (pH 7.5), 60 mM NaCl, and 7 mM MgCl₂ for BamHI, Hind III, PstI, RsaI, HincII, and DraI. Restriction enzymes other than RsaI were obtained from Takara Shuzo, and a restriction enzyme RsaI was obtained from Nippon Genes.

After cleavage with a restriction enzyme, 4 μl of an aqueous solution containing 0.25% bromophenol blue and 50% glycerol was added to the cleavage product, which was then subjected to agarose gel electrophoresis (agarose concentration 0.8 to 2.5%). The agarose was the type II for electrophoresis obtained from Sigma. The electrophoresis buffer was a buffer containing 50 mM Tris-acetate, 80 mM sodium acetate, 72 mM sodium chloride, and 1 mM EDTA. Electrophoresis was carried out using a horizontal gel having a thickness of 5 mm at a voltage of 2 V/cm for 9 to 16 hours. In this electrophoresis, the molecular weight standard was based on a digestion product of the λ phage DNA with restriction enzyme Hind III (Nippon Gene). After the electrophoresis, DNA bands in the agarose gel were stained with a 2 μg/ml ethidium bromide aqueous solution, and radiated with long wavelength ultraviolet light to determine the cleavage pattern. The cleavage patterns obtained by cleavage with a single restriction enzyme separately using various restriction enzymes, and by cleavage with various combinations of two restriction enzymes, were analyzed to determine the relationship between the various restriction enzyme cleavage sites.

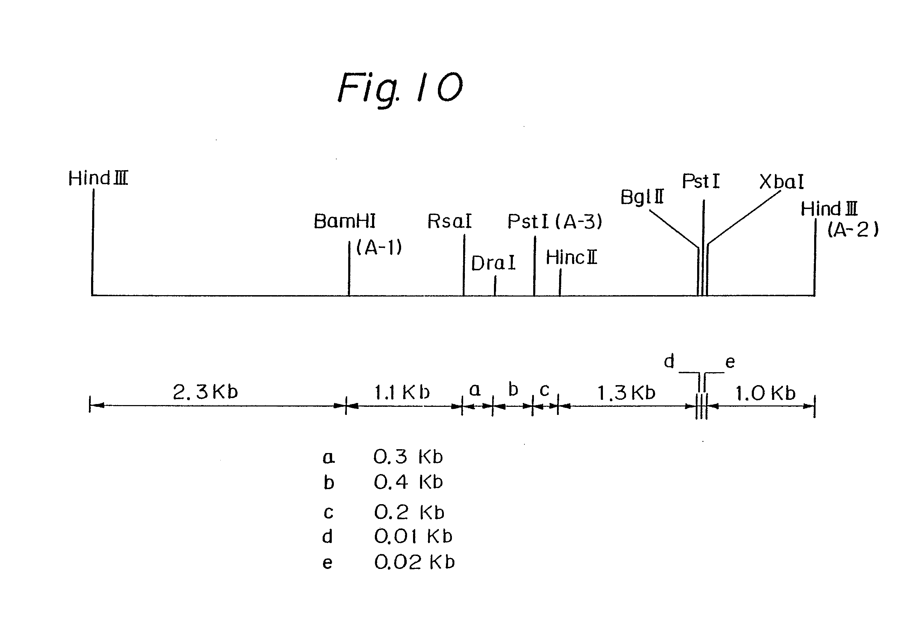

A restriction enzyme cleavage map of the mouse immunoglobulin x chain gene fragment is set forth in Fig. 10. The cleavage sites of DraI, HincII, and RsaI are partially shown.

## Fig. 5   Subcloning of mouse immunoglobulin x chain V-J region gene

Charon 28 phage DNA containing the mouse immunoglobulin x chain V-J region gene was cleaved with Hind III according to the same procedure as described in Example 4, and the cleavage product was subjected to agarose gel electrophoresis. A DNA fragment of 6.5 Kb containing the x chain V-J gene was recovered from the agarose gel by an electro-elution method. On the other hand, 1 μg of an E. coli plasmid pBR322 was cleaved with HindIII according to the same procedure as described in Example 4, 0.5 unit of alkaline phosphatase (E. coli C75; Takara Shuzo) was added to the reaction mixture, and reaction was carried out at 68°C for one hour. After the reaction, the reaction mixture was extracted three times with phenol to inactivate and eliminate alkaline phosphatase. The HindIII/alkaline phosphatase-treated pBR322 solution thus obtained was mixed with a 6.5 Kb HindIII fragment aqueous solution recovered from gel as described above, and after ethanol precipitation, the precipitate was dissolved in a ligation buffer (see, Example 2). To the solution was added 2 units of T4 DNA ligase, and reaction was carried out at 11°C for 12 hours to obtain a hybrid DNA.

The thus-obtained hybrid DNA was used to transform E. coliDHI according to a modified CaCl₂ method (see, M.V. Norgard, K. Keen, J. Monaham, Gene, 3, 279, 1978). That is, an 18-hour culture of E. coli DH-1 was inoculated into 5 ml of L medium (1% trypton, 0.5% yeast extract 0.5% NaCl, pH 7.5) and was grown until the optical density at 600 nm reached 0.3. Cells were washed twice in a cold magnesium buffer (0.1 M NaCl, 5 mM MgCl₂, 5 mM Tris-HCl, pH 7.6, 0°C), the washed cells were resuspended in 2 ml of a cold calcium buffer (100 mM CaCl₂, 250 mM KCl, 5 mM MgCl₂, 5 mM Tris-HCl, pH 7.6, E°C), and the suspension was allowed to stand at 0°C for 25 minutes. The cells were the collected, resuspended in a calcium buffer at a 10-fold concentration in relation to the original concentration, and the concentrated suspension was mixed with the above-mentioned hybrid DNA aqueous solution at a volume ratio of 2:1. The mixture was maintained at 0°C for 60 minutes, 1 ml of LBG medium (1% trypton, 0.5% yeast extract, 1% NaCl, and 0.08% glucose, pH 7.5) were added to the mixture, and culturing was carried out at 37°C for 1 hour. The cultured broth was plated on a selective medium (L medium plate supplemented with 30 μg/ml ampicillin) at a concentration of 100 μl/plate. The plate was incubated at 37°C overnight to allow a growth of transformants. The developed colonies were treated by a known method to prepare DNA, and the target hybrid DNA was confirmed by agarose gel electrophoresis. In this procedure, a DNA fragment of 6.5 Kb containing $V_x$-$J_x$ gene was cloned into a HindIII site of pBR322 to construct a plasmid pYN-MLV1 and pYN-MLV2.

The plasmid wherein the HindIII site (A-2) of the BamHI (A-1) - HindIII (A-2) fragment containing the $V_x$-$J_x$ gene shown in Fig. 10 is positioned near to EcoRI site in the pBR322 is the plasmid pYN-MLV1, and the plasmid containing the BamHI-HindIII fragment in the opposite orientation is the plasmid pYN-MLV2.

The plasmid pYN-MLV1 was digested with BamHI and PstI according to the same procedure as described in Example 4, the digestion product was subjected to agarose gel electrophoresis (agarose concentration 0.7%), and electro-elution was carried out to obtain a BamHI (A-1) - PstI (A-3) DNA fragment shown in Fig. 10. On the other hand, a pUC18 vector (P.L. Biochemicals) was cleaved with BamHI and PstI to obtain a linear vector fragment of about 2.7 Kb. These two DNA fragments were ligated according to the same procedure as described in Example 2, and the reaction mixture was used to transform E. coli JM103 according to the same procedure as described above for E. coli DH1 to obtain transformants. A plasmid DNA was then prepared from the transformant according to a known procedure and a desired plasmid was confirmed by agarose electrophoresis. A pUC18-derived recombinant plasmid pYN-MLVBP containing a part of a gene cloned in Example 3, i.e., BamHI (A-1) - PstI (A-3) fragment shown in Fig. 10, was obtained through this procedure.

### Example 6 Determination of nucleotide sequence

The plasmid pYN-MLV1 described in Example 5 was cleaved with BamHI and PstI according to the same procedure as described in Example 4 to obtain a DNA fragment flanked with BamHI (A-1) and PstI (A-3) shown in Fig. 10. Further, the plasmid pYN-MLV1 was cleaved with BglII and PstI to obtain a DNA fragment of about 1.5 Kb flanked with PstI (A-3) and BglII shown in Fig. 10. These DNA fragments were separately cloned in M13mp18 (P.L. Biochemicals) cleaved with BamHI and PstI. Sequencing was carried out according to the dideoxy chain termination method using an M13 sequencing kit (Takara Shuzo) and [α-³²P]dCTP (Amersham) (see, "DNA Sequence Analysis Manual", ed. M. Takanami and T. Oi, 1983, Kodan Sha, Japan).

The BamHI (A-1) - PstI (A-3) fragment was sequenced in the direction from the PstI end to the 5' end; and the PstI (A-3) - BglII fragment, was sequenced in the direction from the PstI end to the 3' end.

Furthermore, the plasmid pYN-MLVBP was cleaved with RsaI and PstI to obtain a DNA fragment of about 0.65 Kb, which was then cloned in an M13mp19 (P.L. Biochemicals) which has been cleaved with SmaI and PstI. The insert DNA fragment was sequenced in the direction from the RsaI to the 3' end. The digestion with SmaI was carried out in an aqueous solution containing 10 mM Tris-HCl (pH 8.0), 7 mM MgCl₂, 20 mM KCl, and 7 mM 2-mercaptoethanol at 37°C for 2 hours, and then the NaCl concentration was increased to 50 mM for digestion with PstI.

Also, the plasmid pYN-MLVBP was cleaved with RsaI and DraI, or DraI and PstI, and the resulting DNA fragments were fractionated. For fractionation, a 5% acrylamide vertical gel having a thickness of 2 mm was used (see, "Idenshi Sosa Jikken Manual", ed. Y. Takagi, Kodan Sha, Japan). Digestion with RsaI and DraI provided a DNA fragment of about 0.3 Kb; and digestion with DraI and PstI provided a DNA fragment of about 0.4 Kb. The former was ligated to the M13mp18 phage DNA which has been digested with SmaI and dephospholylated according to the procedure as described in Example 5, and the latter was ligated to the M13mp19 phage DNA which has been digested with SmaI and PstI, to obtain a recombinant phage containing a RsaI-DraI DNA fragment and a recombinant phage containing a DraI-PstI (A-3) DNA fragment, both shown in Fig. 10, respectively. The RsaI-DraI fragment was then sequenced in the direction from the DraI end to the 5' end; and the DraI-PstI fragment was sequenced in the direction from the DraI end to the 3' end.

The resulting nucleotide sequence obtained by combining nucleotide sequences of the above-mentioned various DNA fragments is set forth in Fig. 8.

Example 7 Isolation of human chromosomal DNA

$3 \times 10^8$ cells of human cell culture ARH77 were disrupted with a glass bar, and treated with protease K (Sigma) in the presence of 2% SDS, and to the mixture was added phenol saturated with an aqueous solution containing 10 mM Tris-HCl (pH 8.0) and 1 mM EDTA. The whole was centrifuged to separate an aqueous phase and a phenol phase, and the aqueous phase was dialyzed against an aqueous buffer containing 20 mM Tris-HCl (pH 7.5), 100 mM NaCl, and 5 mM EDTA. The dialyzate was treated with ribonuclease A (Sigma), and after extraction with phenol, the aqueous phase was dialyzed against an aqueous buffer containing 10 mM Tris-HCl (pH 8.0) and 1 mM EDTA to obtain about 1.2 mg of human chromosomal DNA.

Example 8 Construction of human gene library

A human chromosomal DNA obtained in Example 7 was cleaved with EcoRI (Takara Shuzo) according the same procedure as described in Example 10, the cleavage product was subjected to agarose gel electrophoresis, and DNA fragments corresponding to 2 to 3 Kb were recovered by the electro-elution method. Next, the DNA fragments were ligated to the λgtWESλB vector (Amersham) according to the same procedure as described in Example 2 to obtain hybrid DNAs wherein a DNA of human origin was inserted between the right arm and left arm of the λgtWESλB vector. The hybrid DNA thus prepared were subjected to in vitro packaging to construct a human gene library (using an Amersham kit).

Example 9 Screening of gene for human immunoglobulin x chain

The gene library in the λgtWESλB phage constructed in Example 8 was infected to E. coli LE392 to form plaques. Clones containing a gene for the human immunoglobuline x chain were selected using as a cross-hybridization probe the $^{32}$P-labeled mouse $C_x$ gene according to the same procedure as described in Example 3. Phage DNA containing the gene for human immunoglobuline x chain was isolated from the selected λgtWESλB phage according to the same procedure as described in Example 3.

Example 10 Preparation of restriction enzyme cleavage map of human x chain C region gene

1 µg of DNA prepared by recloning the phage DNA containing human x chain gene obtained in Example 9 in a plasmid according to the same procedure as described in Example 11 was dissolved in 20 µl of a restriction enzyme cleavage buffer, two units of restriction enzyme (Takara Shuzo) was added to the solution, and digestion was carried out at 37°C for an hour. The digestion product was then subjected to electrophoresis according to the same procedure as described in Example 4 (see, P.A. Hieter, E.E. Max, J.G. Seidman, J.V. Maizel, JR., P. Leder, CELL, 22, 197, 1980).

The above-mentioned restriction enzyme cleavage buffer was a buffer containing 50 mM Tris-HCl (pH 7.5), 7 mM MgCl$_2$, 100 mM NaCl, and 7 mM 2-mercaptoethanol for EcoRI; a buffer containing 10 mM Tris-HCl (pH 7.5), 7 mM MgCl$_2$, 60 mM NaCl, and 2 mM 2-mercaptoethanol for AccI; or a buffer containing 10 mM Tris-HCl (pH 8.0), 7 mM MgCl$_2$, and 7 mM 2-mercaptoethanol for SacI.

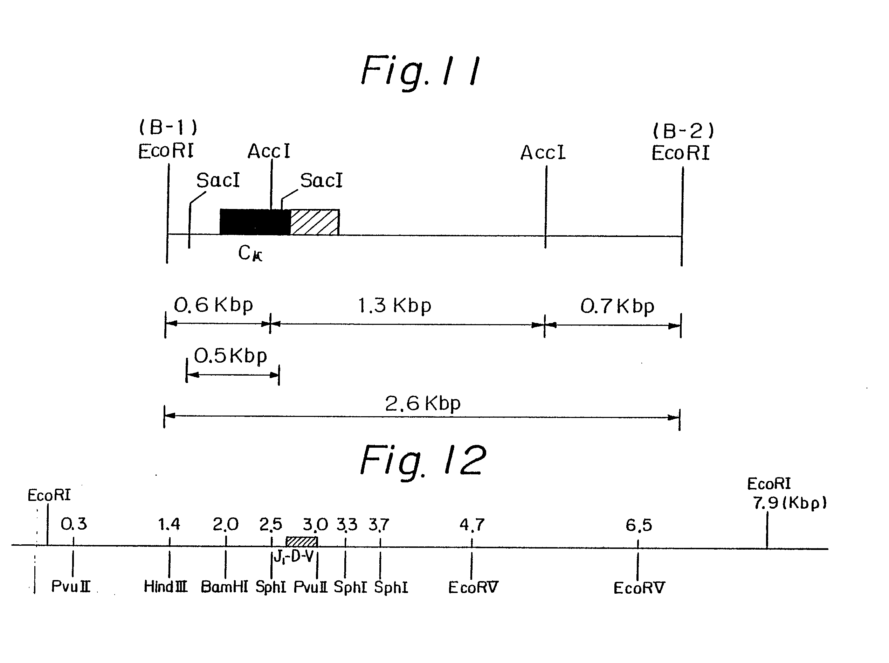

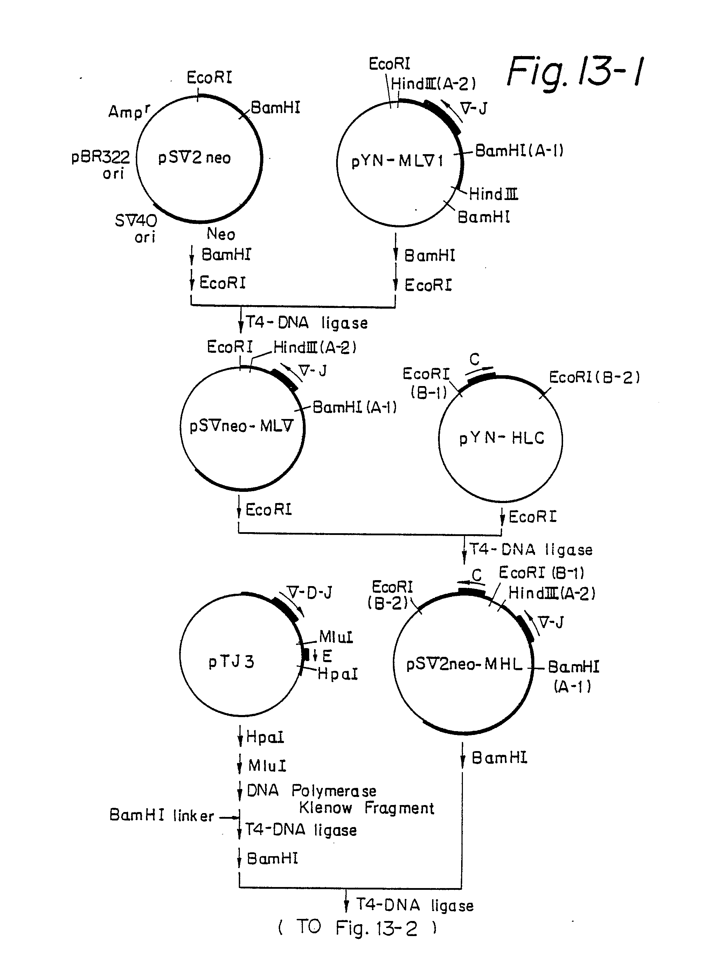

A restriction enzyme cleavage map of the gene containing a coding region for the human immunoglobulin x chain C region is set forth in Fig. 11.

Example 11 Subcloning of gene for human immunoglobulin x chain C region

3 µg of the λgtWESλB phage DNA containing the gene for a human immunoglobulin x chain C region was cleaved with EcoRI according to the same procedure as described in Example 10, the cleavage product was subjected to agarose gel electrophoresis, and a DNA fragment of 2.6 Kb containing $C_x$ gene was recovered from the gel by the electro-elution method. On the other hand, 1 µg of E. coli plasmid pBR322 was cleaved with EcoRI according to the same procedure as described in Example 10, and treated with alkaline phosphatase according to the same procedure as described in Example 5. The EcoRI-cleaved and alkaline phosphatase-treated pBR322 DNA thus obtained was mixed with the above-mentioned 2.6 Kb EcoRI DNA fragment recovered from the gel, and after ethanol precipitation, the resulting precipitate was dissolved in 10 $\mu$l of ligation buffer (see, Example 2), and ligation was carried out using a T4 DNA ligase according to the same procedure as described in Example 5.

The ligation mixture was used to transform E. coli DH1, and a target hybrid DNA was isolated from the transformant and confirmed.

## Example 12 Construction of mouse-human chimera antibody gene

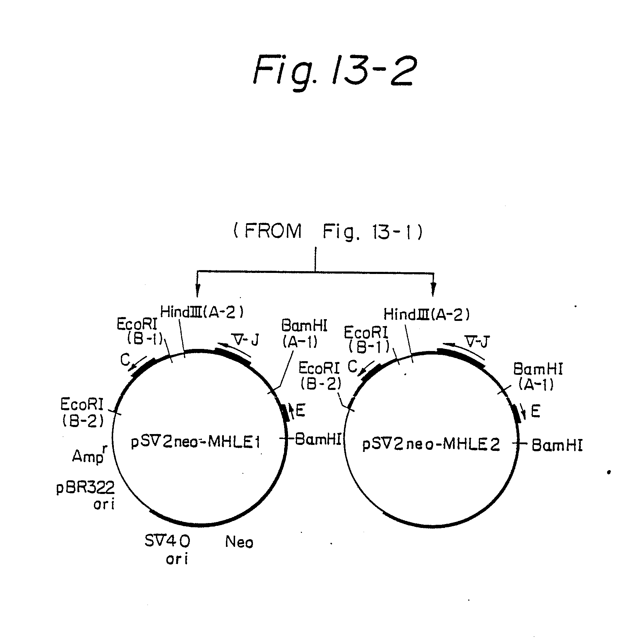

The plasmid pYN-MLV1 containing the gene for the human immunoglobulin x chain V-J region was cleaved with BamHI and EcoRI according to the same procedures as described in Examples 4 and 10, and the cleavage product was subjected to agarose electrophoresis. The resulting EcoRI-BamHI fragment of 4.2 Kb containing the $V_x$-$J_x$ gene was inserted into the EcoRI-BamHI site of the pSV2neo vector (see R.J. Southern and P. Berg, J. Mol. Appl, Genet., 1, 327, 1982) to construct a plasmid pSV2neo-MLV. The construction process is set forth in Fig. 13-1.

Next, a plasmid pYN-HLC containing the gene for the human immunoglobulin x chain C region, obtained in Example 11 was cleaved with EcoRI according to the same procedure as described in Example 10, and the cleavage product was subjected to agarose gel electrophoresis. A DNA fragment of 2.6 Kb containing the $C_x$ gene thus obtained was inserted into the EcoRI site of the above-mentioned plasmid pSV2neo-MLV according to the same procedure as described in Example 11. Among the resulting clones, a clone containing a plasmid wherein the EcoRI (B-1) end of the EcoRI (B-1) - EcoRI (B-2) fragment containing the human C $_x$ gene is present near to the mouse $V_x$-J $_x$, so that the direction of the reading frame of mouse V $_x$-$J_x$ gene conforms to that of the human $C_x$ gene, were selected, and the selected plasmid was described as pSV2neo-MHL. The construction process is set forth in Fig. 13-1.

On the other hand, a plasmid pTJ3 containing the human immunoglobulin $\gamma_1$ gene described in Japanese Unexamined Patent Publication No. 60-120991 was cleaved with MluI and HpaI according to the procedure described in Example 10, except that the cleavage buffer was an aqueous buffer containing 10 mM Tris-HCl (pH 7.5), 7 mM MgCl$_2$, .

150 mM NaCl, and 7 mM 2-mercaptoethanol for MluI cleavage. After cleavage with MluI and recovery of the DNA fragment by ethanol precipitation, the recovered DNA was cleaved with HpaI in a cleavage buffer containing 10 mM Tris-HCl (pH 7.5), 100 mM KCl, 7 mM MgCl$_2$, and 7 mM 2-mercaptoethanol. After agarose gel electrophoresis, a MluI-HpaI fragment of about 0.9 Kb containing the human $\gamma_1$ chain gene enhancer region was obtained. This DNA fragment was dissolved in a polymerase buffer containing a 37 mM potassium phosphate, 6.7 mM MgCl$_2$, and 1 mM 2-mercaptoethanol (pH 7.4), and to the solution was added a Klebnow fragment of DNA polymerase (New England Biolabs) in the presence of dNTP to blunt the ends. Then, a BamHI linker (Takara Shuzo) was linked to the ends and the BamHI linker was cleaved with BamHI, and a BamHI fragment containing human $\gamma_1$ chain gene enhancer region was obtained by agarose gel electrophoresis. This DNA fragment was inserted into the BamHI site of the above-mentioned plasmid pSV2neo-MHL to construct different plasmids pSV2neo-MHLE1 and pSV2neo-MHLE2. The construction process of these plasmids is set forth in Figs. 13-1 and 13-2.

## Example 13 Construction of mouse gene library

150 $\mu$g of the mouse chromosomal DNA obtained in Example 1 was completely digested with EcoRI (Takara Shuzo) according to the same procedure as described in Example 10, the digestion product was subjected to agarose gel electrophoresis, and 5 $\mu$g of a DNA fragment corresponding to 7 to 9 Kb was extracted from the agarose gel.

Next, 0.4 $\mu$g of this DNA was linked with the Charon 4A vector EcoRI arm (Amersham) according to the same procedure as described in Example 2, and in vitro packaging was carried out to obtain an 8 x 10$^6$ PFU/$\mu$g mouse NL-1 gene library.

## Example 14 Screening of mouse antibody H chain gene

The DNA derived from mouse NL-1 cell line obtained in Example 13 was infected to E. coli LE392 to form plaque. Clones containing the mouse antibody H chain gene were selected by the $^{32}$P-labeled mouse antibody H chain J gene according to the same procedure as described in Example 3.

In this manner, a gene containing an entire V region of 7.9 Kb (5' flanking region, VDJ region, and enhancer region) was isolated.

## Example 15 Preparation of restriction enzyme cleavage map of mouse antibody H chain gene

An EcoRI fragment of 7.9 Kb containing the H chain gene derived from the mouse NL-1 cell line, obtained in Example 14, was subcloned in a plasmid pBR322 according to the same procedure as described in Example 5, and a recombinant plasmid pBR-NL-1-H wherein this H chain gene was inserted into the EcoRI site of pBR222 was prepared by a known method. The plasmid pBR-NL-1-H was cleaved with a restriction enzyme, and a restriction enzyme cleavage map was made in the same manner as described in Example 4. Cleavage with BamHI and Hind III were carried out according to the procedure described in Example 4; and cleavage with EcoRI was carried out according to the procedure described in Example 10. In the PvuII-cleavage, an aqueous buffer containing 10 mM Tris-HCl (pH 7.5), 7 mM $MgCl_2$, 60 mM NaCl, and 7 mM 2-mercaptoethanol was used as a cleavage buffer; and in the EcoRV and SphI cleavages, an aqueous buffer containing 50 mM Tris-HCl (pH 7.5), 150 mM NaCl, 7 mM $MgCl_2$, and 7 mM 2-mercaptoethanol was used. The cleavage reaction was carried out in the presence of 1 μg of a plasmid DNA and 2 units of a restriction enzyme at 37°C for at least 2 hours. The restriction enzyme map thus prepared is set forth in Fig. 12.

## Example 16 Determination of nucleotide sequence of mouse antibody H chain VDT region gene

The above-mentioned plasmid pBR-NL-1-H was cleaved with restriction endonucleases SphI and PvuII to obtain two DNA fragments flanked by SphI and PvuII ends which were provided by the inserted gene fragment. These two fragments were cloned to M13 phage vectors mp18 and mp19 (P.L. Biochemicals) which had been cleaved with SphI and HincII. Sequencing was carried out using an M13 sequencing kit (Takara Shuzo) by a dideoxy chain termination method (see M. Takanami and T. Oi, Example 6). A 5' fragment of the SphI-PvuII fragment was sequenced from the SphI site to the 3'-direction and from the PvuII to the 5'-direction, and a 3' fragment was sequenced from the PvuII site to the 3'-direction. The nucleotide sequence of the V region gene is set forth in Fig. 5.

## Example 17 Determination of nucleotide sequence of human antibody H chain C region gene

The nucleotide sequence of the human antibody H chain C region gene was determined by the Maxam-Gilbert method (see A. Maxam and W. Gilbert, Methods Enzymol., 65, 499, 1980).

The plasmid pTJ5 described in Japanese Patent Publication No. 60-145089 was digested with PstI, and labeled with [α-$^{32}$P]ddATP using a 3'-end labeling kit (Amersham). A $^{32}$P-labeled DNA was digested with SmaI, and a desired DNA fragment was separated by polyacrylamide gel electrophoresis (gel concentration 5%) and extracted from the gel. The $^{32}$P-labeled PstI-SmaI fragment thus obtained was subjected to nucleotide-specific partial digestion for each nucleotide, and the resulting DNA fragments were separated by polyacrylamide gel electrophoresis (gel concentration 8 to 23%). Autoradiography at -80°C was carried out for 2 to 7 days, and the digestion pattern then analyzed. Other DNA fragments were labeled at their 3'-end with [α-$^{32}$P] ddATP, or at their 5'-end with [α-$^{32}$P]ATP, and sequenced by Maxam-Gilbert method. The results for each of the DNA fragments were combined to determine the entire nucleotide sequence, which is set forth in Fig.2.

## Example 18 Construction of Plasmid pMH1 for expression of chimera antibody gene

The plasmid pBR-NL-1-H obtained in Example 15 was cleaved with EcoRV and BamHI according to the same procedure as described in Examples 4 and 15 to form an EcoRV-BamHI fragment of about 2.7 Kb containing the entire H chain V region, which was then separated and recovered by electro-elution according to the same procedure as described in Example 4.

On the other hand, a plasmid vector pSV2gpt (see R.C. Mulligan and P. Berg, Proc. Natl. Acad. Sci., USA, 78, 2072, 1981) was cleaved with BamHI and HpaI according to the same procedures described in Examples 4 and 15, and a linear vector of about 4.5 Kb containing an Eco gpt gene was isolated. This linear vector is designated as "fragment-1".

An EcoRV-BamHI fragment of about 2.7 Kb derived from the pBR-NL-1-H prepared as described above was ligated with the above-mentioned BamHI-HpaI fragment-1 derived from pSV2-gpt, using a T4 DNA ligase according to the same procedure as described in Example 5, and the ligation product was cleaved with BamHI to obtain a linear vector gene of about 8.0 Kb containing a V region gene of about 2.7 Kb derived from the NL-1-H, and the linear vector gene was separated and purified. This vector gene is designated as "fragment-2".

On the other hand, a plasmid pSV2-HIG1 containing an entire gene (about 21 Kb) for human immunoglobulin $\gamma_1$ chain, described in Japanese Unexamined Patent Publication No. 60-145089, was cleaved with MluI according to the same procedure as described in Example 12 to obtain a DNA fragment of about 17 Kb containing a human enhancer and $C_\gamma 1$ gene. To this fragment was attached a BamHI linker (Takara Shuzo) to form a DNA fragment flanked with BamHI sites. This fragment is designated as "fragment-4".

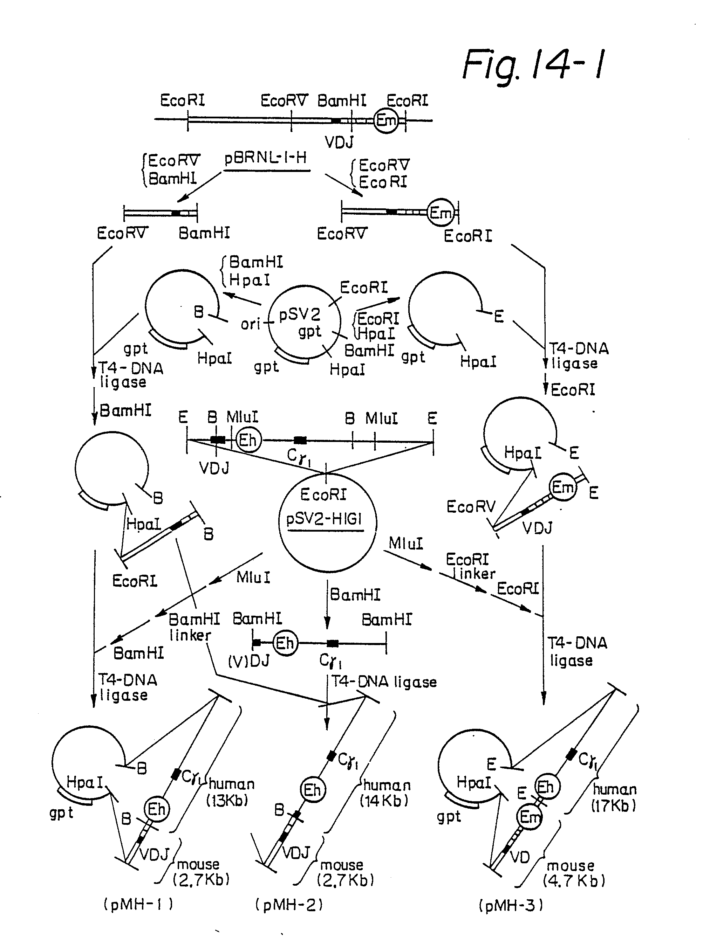

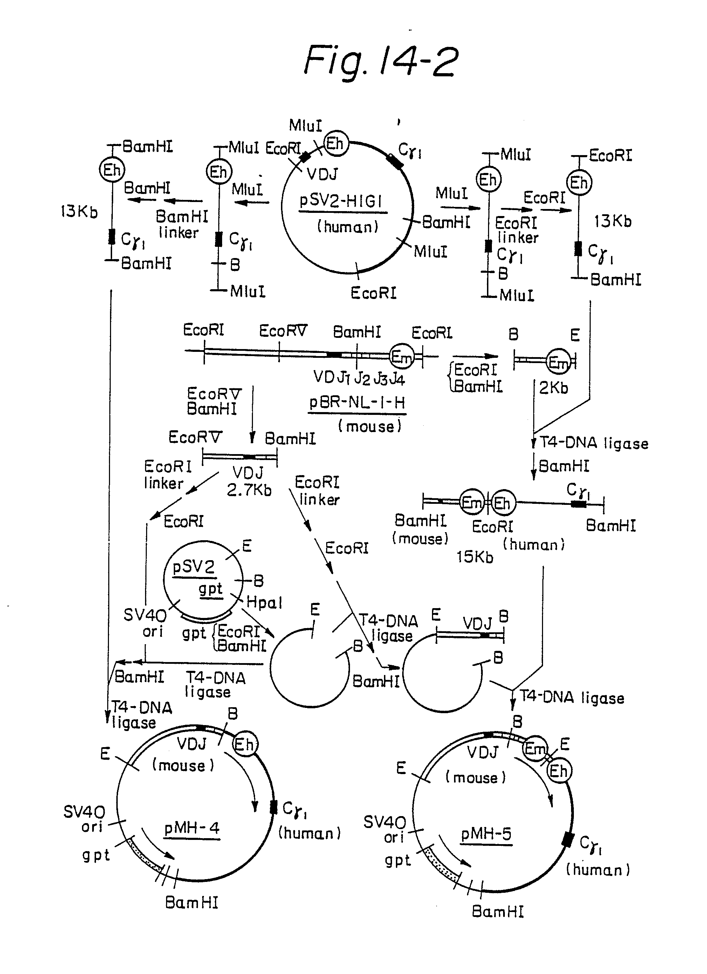

The fragment-4 thus prepared was ligated with the above-prepared fragment-2 using a T4 DNA ligase, and the ligation product was used the transform E. coli MC1000. A plasmid containing a chimera antibody gene wherein the V region of mouse origin is linked with the C region of human origin via a human enhancer (Eh) was obtained by this procedure. This plasmid is designated as pMH-1. The construction process of this plasmid pMH-1 is set forth in Fig. 14-1.

## Example 19 Construction of plasmid pMH-2 for expression of chimera antibody gene

Plasmid pSV2-HIG1 containing a human antibody H chain gene of about 21 Kb, described in Japanese Unexamined Patent Publication No. 60-145089, was cleaved with a restriction enzyme BamHI and a DNA fragment of about 14 Kb containing a human enhancer (Eh) and $C_\gamma 1$ gene. This fragment was ligated with the above-mentioned fragment-2 using a T4 DNA ligase, and the ligation product was used to transform E. coli MC1000. A plasmid containing a chimera antibody gene wherein the V region of mouse origin is linked with the C region of human origin via a human enhancer was obtained by this procedure. This plasmid is designated as pMH-2. The construction process of the plasmid pMH-2 is set forth in Fig. 14-1.

## Example 20 Construction of plasmid pMH-3 for expression of chimera antibody gene

The plasmid pBR-NL-1-H described in Example 15 was cleaved with EcoRV and EcoRI to obtain a DNA fragment of about 4.7 Kb containing an entire mouse H chain V region gene, and a mouse enhancer (Em) was obtained according to the same procedure as described in Example 15.

Next, the above-mentioned vector pSV2-gpt was cleaved with EcoRI and HpaI to obtain a DNA fragment of about 4.5 Kb containing an Eco gpt gene, which fragment was purified. This fragment was ligated with the above-mentioned DNA fragment of about 4.7 Kb containing the mouse V region gene, using a T4 DNA ligase, and the ligation product was cleaved with EcoRI to obtain a linear DNA fragment of about 9.2 Kb containing a V region gene of about 4.7 Kb derived from NL-1-H, which DNA fragment was purified. This fragment is designated as "fragment-3".

On the other hand, to a DNA fragment of about 17 Kb containing a human enhancer (Eh) and $C_\gamma 1$ gene and prepared according to the same procedure as described in Example 18, was attached an EcoRI linker (Takara Shuzo) to form a DNA fragment flanked with EcoRI sites. This fragment is designated as "fragment-5".

This fragment-5 was ligated with the above-mentioned fragment-3 using a T4 DNA ligase to obtain a plasmid containing a chimera antibody gene wherein the V region of mouse origin is linked with the C region of human origin via a mouse enhancer and a human enhancer. This plasmid is designated as pMH-3. The construction process of the plasmid pMH-3 is set forth in Fig. 14-2.

## Example 21 Construction of plasmid pMH-4 for expression of chimera antibody gene

The plasmid pBR-NL-1-H described in Example 15 was cleaved with EcoRV, and to the cleavage product was attached an EcoRI linker (Takara Shuzo) according to the same procedure as described in Example 12, and the resulting fragment was cleaved with EcoRI and BamHI to obtain an EcoRV-BamHI fragment whose EcoRV site was converted to EcoRI site with EcoRI linkers, which fragment contains the entire V region gene and has a length of about 2.7 Kb.

On the other hand, the above-mentioned vector pSV2-gpt was cleaved with EcoRI and BamHI to form a linear vector of 4.6 Kb containing the Eco gpt gene. The linear vector was then separated and purified. This linear vector was ligated with the above-mentioned EcoRI-BamHI fragment of about 2.7 Kb using a T4 DNA ligase, and the ligation product was cleaved with BamHI to obtain a vector DNA fragment of about 7.3 Kb containing the V region gene derived from NL-1-H. This fragment is set forth as "fragment-6".

This fragment-6 was ligated with the above-mentioned fragment-4 containing the human enhancer (Eh) and $C_\gamma 1$ gene using a T4 DNA ligase, and the reaction mixture was used to transform E. coli MC1000. A plasmid containing a chimera antibody gene wherein the V region of mouse origin gene is linked with the C region gene via a human enhancer (Eh) was obtained by this procedure. This plasmid is designated as pMH-4. The construction process of the plasmid pMH-4 is set forth in Fig. 14-2.

### Example 22 Construction of plasmid pMH-5 for expression of chimera antibody gene

The plasmid pBR-NL-1-H described in Example 15 was cleaved with BamHI and EcoRI to form a DNA fragment of about 2 Kb containing a mouse enhancer, which fragment was then separated and purified. This fragment of about 2 Kb was ligated with the fragment-5 obtained in Example 20 using a T4 DNA ligase, and the ligation product was cleaved with BamHI to obtain a BamHI fragment of about 13 Kb containing mouse enhancer and human enhancer, as well as the human $C_\gamma 1$ gene. This BamHI fragment was ligated with the fragment-6 obtained in Example 21 using a T4 DNA ligase, and the ligation product was used to transform E. coli MC1000. A plasmid containing a chimera antibody gene wherein the V region of mouse origin is linked with the C region of human origin via a mouse enhancer and human enhancer was obtained by this procedure. This plasmid is designated as pMH-5. The construction process of the plasmid pMH-5 is set forth in Fig. 14-2.

### Example 23 Expression of chimera antibody H chain gene

Each of chimera antibody expression plasmid pMH-1, pMH-2, pMH-3, pMH-4, and pMH-5 was used to transform E. coli MC1000 according to the same procedure as described in Example 5, to obtain transformants. The transformant was then cultured in an ampicillin-containing medium, and in a chloramphenicol-containing medium to amplify the plasmid. The cultured E. coli cells were treated with lysozyme (Sigma) to prepare protoplasts. The protoplasts thus obtained were fused with 2 x 10⁶ cells of a mouse myeloma J558L cell line (λ producing strain) or mouse myeloma cells of a X63Ag8.653 cell line (non-antibody producing strain) in 0.5 ml of a fusion medium containing 50% PEG 4000 at a room temperature for 2 to 7 minutes, the fusion mixture was then diluted with an MEM medium (Gibco, Grand Island, NY), and the whole was centrifuged to eliminate PEG 4000. Cells were grown in the RPMI 1640 complete medium (Gibco, Grand Island, NY) at 37°C for 48 to 72 hours. Cell surfaces of transformed J558L cells were fluorescence-stained using FITC-labeled goat anti-human IgG (Cappel Laboratories Inc.) to test the expression of the chimera antibody gene. As a result, it was found that cells containing the expression plasmid pMH-1, pMH-2 or pMH-4 provide the gene expression at a high level, and cells containing the expression plasmid pMH-3 or pMH-5 provide the gene expression at a relatively low level.

The above-mentioned expression plasmid pMH-4 was introduced into mouse myeloma J558L cells or X63Ag8.653 cells by protoplast fusion, and the transformed cells were transferred to a selection medium containing 250 μg/ml xanthine, 15 μg/ml hypoxanthine, and 6 μg/ml mycophenolic acid. After a daily exchange of medium for two days and culturing for an additional 10 to 25 days, a mycophenolic acid-resistant transformant was obtained. These transformants derived from the J558L cells and X63Ag8.653 cells are designated as J558L-H and X63Ag8.653-H, respectively.

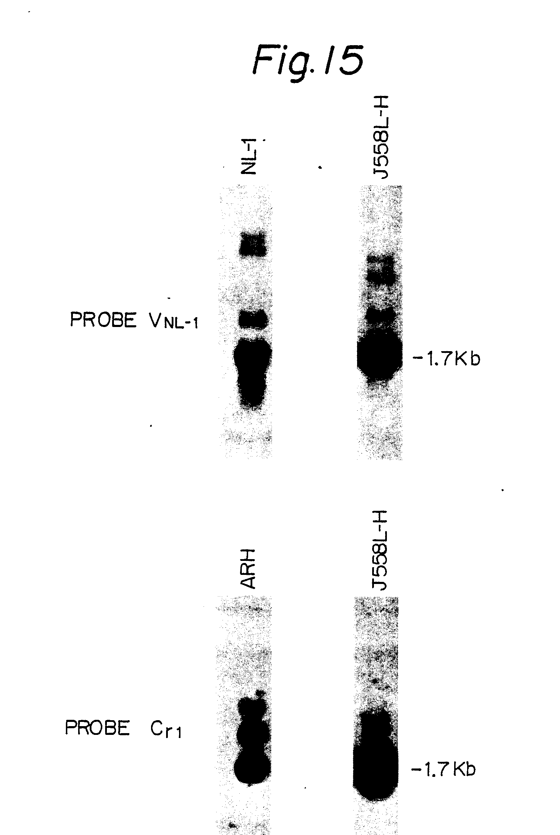

RNA was extracted from the transformant cells thus obtained, and 20 μg each of the extracted RNA was subjected to Northern hybridization (see T. Maniatis et al. Molecular Cloning, Cold Spring Harbor Lab., 1982). As probes, a BamHI-PvuII DNA fragment which contains the mouse V-D-J gene and was derived from a plasmid pBR-NL-1-H, and a PstI (2) - PstI (3) DNA fragment which contains a part of the human $C_\gamma 1$ gene and was derived from a plasmid pSV2-HIGI, both labeled with ³²P, were used. As seen from an autoradiogram obtained by using J558L-H cells shown in Fig. 15, a band corresponding to the mRNA for entire secretion-type H chain was observed at a position of 1.7 Kb.

### Example 24 Analysis of expression product of chimera antibody H chain gene

10⁷ cells of the J558L-H cell line (λ producing strain) transformed with the chimera antibody H chain gene described in Example 23 were washed with a phosphate buffered (pH 7.5) saline, and suspended in methionine-free RPMI 1640 supplemented with 10% setal calf serum, and after the addition of 300 μCi of [³⁵S]-methionine, cultured at 37°C for 8 hours. In this manner, the produced proteins were biosynthetically labeled with ³⁵S, and a supernatant was recovered. A produced antibody was recovered from the supernatant by immuno-precipitation. The recovered antibody was reduced by using 2-mercaptoethanol, to put it under a condition allowing a separation of the H chain and L chain. The reduced sample was then separated by SDS-polyacrylamide gel electrophoresis, and the gel was dried and subjected to autoradiography (see Denki Eido Gakkai ed., Denki Eido Jikken Ho, Bunko Do, Japan, 1976).

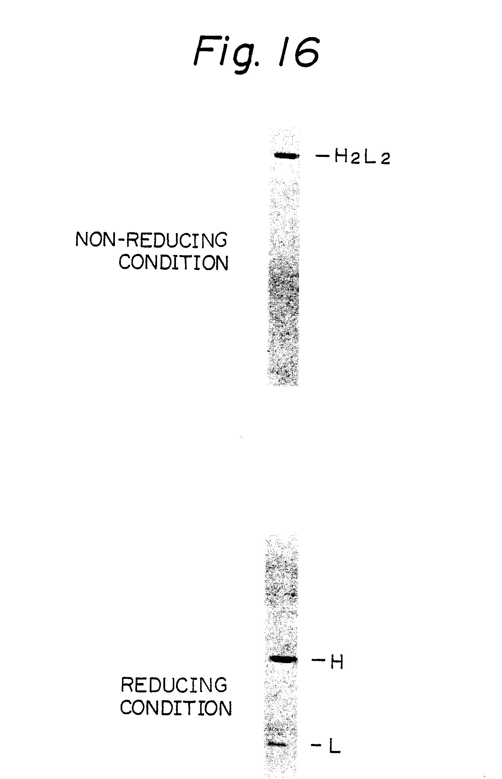

It was confirmed that the expression product of chimera H chain gene artificially introduced was extra-cellularly secreted in the form of a conjugation with a product of the λ chain gene endogeneously present in J558L cells. The autoradiogram is shown in Fig. 16.

## Example 25 Expression of chimera antibody L chain

To J558L-H cells and X63Ag8.653-H cells prepared in Example 23, plasmids pSV2neo-MHLE1 or pSV2neo-MHLE2 were introduced according to the DEAE-dextran method (see J. Benarji, L. Olson, W. Schaffner, Cell, 33, 729, 1983). That is, to $10^7$ cells was added a mixture of 80 μg of plasmid DNA and DEAE-dextran (Pharmacia), and after incubation, the mixture was transferred into an RPMI 1640 complete medium (Flow Laboratories) supplemented with 10% fetal calf serum. A drug resistant strain was selected by a half-volume exchange of an RPMI 1640 complete medium containing 1 to 15 mg/ml of Geneticin G418 (Gibco). After 10 to 20 days of culturing, grown cells were selected as stable transformants. The transformants derived from J558L-H and X63Ag8.653-H are designated as J558L-HL and X63Ag8.653-HL, respectively.

## Example 26 Analysis of mRNA from cells transformed with chimera antibody L chain gene

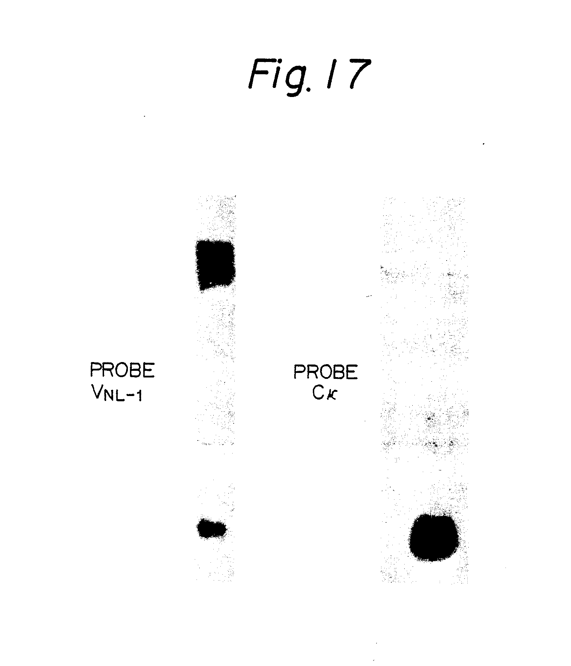

From J558L-HL cells and X63Ag8.653-HL cells prepared in Example 25, RNA was extracted and analyzed for a transcription product of the chimera L chain gene according to the same procedure as described in Example 23. As hybridization probes for the chimera L chain gene, a PstI (A-3) - HincII DNA fragment shown in Fig. 10 in the plasmid pYN-MLV-1 was used for the V region probe, and a ScaI (B-1) - ScaI (B-2) DNA fragment in the plasmid pYN-HLC, both labeled with $^{32}$P, was used. An autoradiogram obtained by using J558L-H cells shown in Fig. 17. As shown in Fig. 17, both probes provided bands at the same position.

## Example 27 Analysis of chimera antibody L chain expression product by polyacrylamide gel electrophoresis

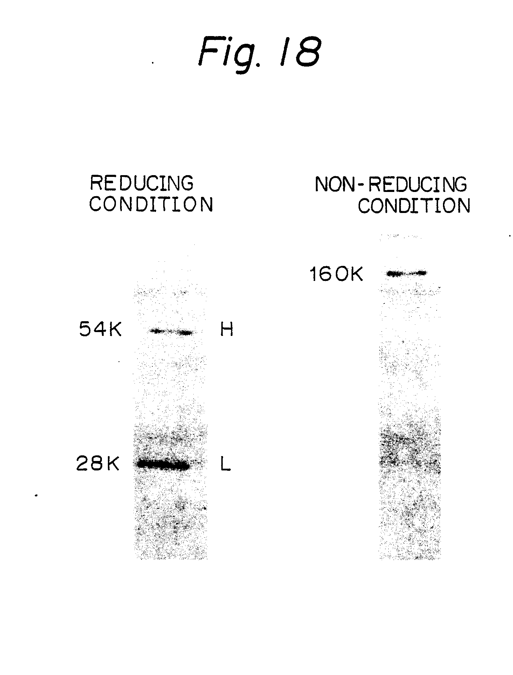

$10^{11}$ cells of X62Ag8.653-HL prepared in Example 25 were washed with a phosphate buffered (pH 7.0) saline and suspended in a methionine-free RPMI 1640 medium supplemented with 10% fetal calf serum, and after the addition of 300 μCi of [$^{35}$S]-methionine, were cultured for 8 hours. In this manner, the produced proteins were biosynthetically labeled with $^{35}$S, and a supernatant was recovered. The produced antibody was immunoprecipitated and the antibody was reduced with 2-mercaptoethanol, to put it under a condition allowing separation of the H chain and L chain. After the reduced mixture was separated by SDS-polyacrylamide gel electrophoresis, the gel was dried and subjected to autoradiography (see Denki Eido Gakkai ed., Denkieido Jikken Ho, Bunko Do, 1976). The production of both the H chain protein and L chain (x chain) protein was observed as shown in Fig. 18. Further, it was shown that the chimera antibody L chain (x chain) protein expressed by the chimera antibody L chain (x chain) gene later introduced was secreted together with the chimera antibody H chain (γ1) expressed by the chimera antibody H chain gene previously introduced in the form of a conjugate thereof, because an experiment under a non-reducing condition provided a band at a position of 160 K.

## Example 28 Assay for antigen binding specificity of chimera antibody molecule

The X63Ag8.653-HL prepared by transforming non-antibody producing parent cells X63Ag8.653 with both the chimera antibody H chain gene and chimera antibody L chain gene as described in Example 24 was cultured in an RPMI 1640 complete medium. A supernatant from the culture was assayed to determine the antigen binding specificity of the produced chimera antibody. As target cells, Manca cells expressing the human common acute lymphocytic leukemia common antigen were used. The Manca cells were cultured in an RPMI 1640 complete medium, and $1 \times 10^6$ of the cultured cells were collected and washed with a phosphate buffered (pH 7.0) saline. To the washed cells was added 1 ml of a supernatant from X63Ag8.653-HL culture prepared as described above. A reaction was carried out on ice for 30 minutes. After the non-absorbed antibody was washed off, Monca cells on which the chimera antibody was bound were fluorescence-stained using FITC-labeled goat anti-human γ chain antibody for observation under a fluorescence microscope. The Manca cells treated with X63Ag8.653-HL culture supernatant emitted a fluorescence revealing that the chimera antibody contained in the X63Ag8.653-HL culture supernatant is bound to the Manca cells, i.e., that the chimera antibody has a specificity to the human common acute lymphocytic leukemia antigen. A similar result was obtained for a J558L-HL culture supernatant. X63Ag8.653 cells, J558L cells, as well as J558L-H, and X63Ag8.653-H transformed with the chimera antibody H chain gene alone, provided negative results.

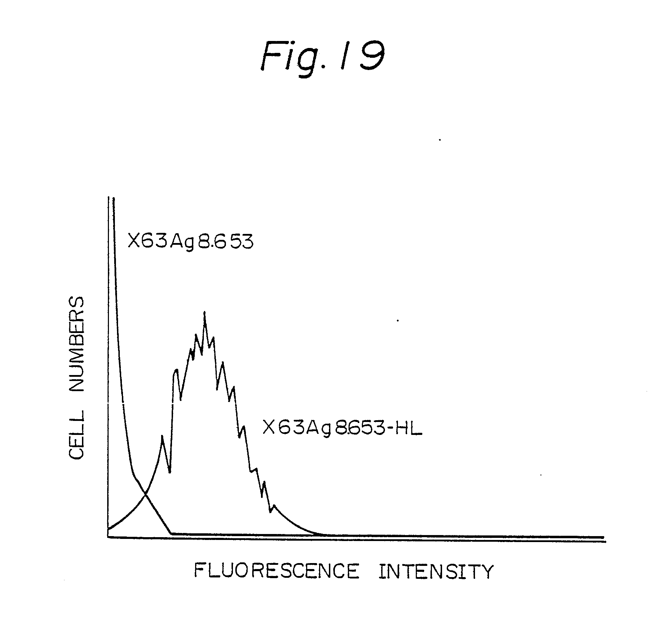

Further, Manca cells treated by the same method as described for fluorescent microscopy were analyzed by flow cytometry (by Coulter EPICS-V) measuring FITC fluorescence intensity. Supernatants from the J558L-HL culture and X63Ag8.653-HL culture provided positive results, although supernatants from the J558L culture, X63Ag8.653 culture, J558-H culture, and X63Ag8.653-H culture provided negative results. The results for the X63Ag8.653-HL culture are shown in Fig. 19.

Example 29 Complement-dependent cytotoxicity test

It is known that a kind of antibody IgG causes cytotoxicity against target cells through the activation of complements (E.J. Brown, K.A. Joiner and M.M. Frank, "Complement" in Fundamental Immunology, p 645, W.E. Paul ed., Raven Press, 1984). The chimera antibody of the present invention was tested for cytotoxicity using Monca cells labeled with $^{51}$Cr.

Transformant X63Ag8.653-HL cells described in Example 25 were cultured in an RPMI 1640 complete medium, and about 70 ml of culture supernatant applied to a protein A sepharose column to recover the chimera antibody, which was then subjected to the test.

On the other hand, $10^7$ Manca cells were incubated in an RPMI 1640 complete medium containing 100$\mu$Ci of $^{51}$Cr-chromic acid at 37°C for one hour to label them with $^{51}$Cr (see S. Hinuma, M. Tada and K. Kumagai, "Assay of complement-depending cell mediated cytotoxicity using $^{51}$Cr-labeled cells" in Men-eki Jikken Sosa Ho, Vol VIII, p 2433, 1979, Japanese Society for Immunologist).

After washing off residual $^{51}$Cr-chromic acid, the washed cells were mixed with the chimera antibody and complement (prepared from rabbit). The mixture was then incubated at 37°C for 30 to 60 minutes to release $^{51}$Cr, the amount of which was used as a measure of the cytotoxicity. An addition of both the chimera antibody and the complement provided an 84% $^{51}$Cr release (84% cytotoxicity) compared with cells incubated alone, although the chimera antibody alone exhibited 9.0% and the complement alone showed 22% cytotoxicity, revealing a complement-dependent cytotoxicity of the chimera antibody.

Example 30 Antibody-dependent cell-mediated cytotoxicity test

The antibody-dependent cell-mediated cytotoxicity (ADCC) was determined by cytotoxicity against Manca cells in the presence of human effector cells and an antibody (see I. Hellstroem, V. Brankovan and K.E. Hellstroem, Proc. Natl. Acad. Sci. USA, 82, 1499, 1985).

2 x $10^6$ Manca cells were labeled with 100$\mu$Ci of [$^{51}$Cr]-chromic acid, and 2 x $10^4$ of the labeled cells were used for one ADCC test.

Effector cells were prepared from the peripheral blood of a healthy person using a lymphocyte separating solution (Bionetics Laboratory Products).

Transformant X63Ag8.653-HL cells described in Example 25, as well as mouse hybridoma NL-1 cells as a positive control, were cultured in an RPMI 1640 complete medium to obtain a supernatant. The antibody contained in about 500 $\mu$l of the supernatant was used for one experiment.

2 x $10^4$ labeled Manca cells, $10^6$ human effector cells, and 50 $\mu$l of antibody solution were mixed, and the mixture was incubated at 37°C for 6 hours in a 5% carbon dioxide atmosphere. The release of $^{51}$Cr into supernatant was determined as a measure of the cytotoxicity. As a parallel run, Manca cells alone were incubated under the same conditions to determine the release of $^{51}$Cr as a reference measurement. The reference measurement was 9 to 12%, and used to correct the test measure. The results obtained were an average of three measurements.

A test using the present human-mouse chimera antibody produced by X63Ag8.653-HL provided a 24.5% cytotoxicity; and a test using NL-1 mouse antibody as a positive control provided a 13.8% cytotoxicity. On the other hand, a test without an antibody provided a 2.3% cytotoxicity; and a test without effector cells (chimera antibody alone) provided a 0.9% measurement.

Example 31 Therapeutic experiment using experimental animals

Tumors of an appropriate size were observed about 2 to 3 weeks after $10^7$ Manca cells were injected subcutaneously into the backs of Balb/c nude mice (obtained from Kyudo). The tumor-carrying mice thus prepared were used for the following therapeutic experiments.

The chimera antibody prepared as described in Example 29 was directly injected to the tumor of the above-mentioned tumor-carrying mice in an amount of about 20 $\mu$g/mouse. After about two weeks from the injection, it was clearly observed that necrosis expanded within the tumor. Moreover, in a comparison of the size of all tumors, the tumor size of mice to which the chimera antibody was injected was smaller than that of mice to which a non-specific mouse antibody as a control was injected, revealing the effectiveness of the chimera antibody topically injected into the tumor.

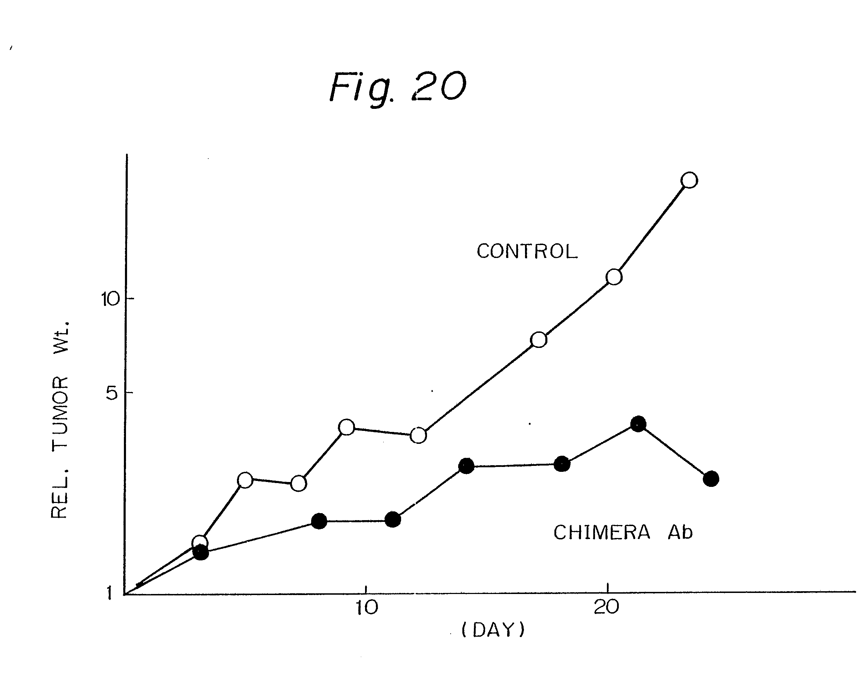

Moreover, the effectiveness of the systemically introduced chimera antibody was confirmed by sequential intraperitoneal injections of the antibody to the tumor-carrying mice. By four sequential injections at the first, third, eighth, and 21st days, a repression of the tumor growth was observed, revealing the effectiveness of systemic application of the present chimera antibody. Figure 20 shows the result of a systemic application of the chimera antibody, compared with that of the non-specific mouse antibody. In the figure, the relative tumor weight is plotted against the time in days.

Reference Example 1.    Isolation of human chromosomal DNA

$3 \times 10^8$ cells of human cell culture ARH77 were disrupted with a glass bar, and treated with protease K (Sigma) in the presence of 2% SDS, and to the mixture was added phenol saturated with an aqueous solution containing 10 mM Tris-HCl (pH 8.0) and 1 mM EDTA. The whole was centrifuged to separate an aqueous phase and a phenol phase, and the aqueous phase was dialyzed against an aqueous buffer containing 20 mM Tris-HCl (pH 7.5), 100 mM NaCl, and 5 mM EDTA. The dialyzate was treated with ribonuclease A (Sigma), and after extraction with phenol, the aqueous phase was dialyzed against an aqueous buffer containing 10 mM Tris-HCl (pH 8.0) and 1 mM EDTA to obtain about 1.2 mg of human chromosomal DNA (see N. Blim, D.W. Stafford, Nucleic Acids Research 3, 2 303, 1976).

Reference Example 2.    Construction of human gene library

150 μg of the chromosomal DNA obtained in Reference Example 1 was digested with a restriction enzyme EcoR I (Takara Shuzo, Japan) according to the same procedure as later described in Reference Example 4, and the digestion product was subjected to sucrose density-gradient centrifugation (sucrose concentration 10 - 40%, wt/vol; 28000 rpm, 120000Xg; 15 hours; 20°C) to obtain 4.3 μg of DNA fragments corresponding to 15 to 23 Kb.

Next, 0.80 μg of the DNA fragment thus obtained were ligated with the Charom 4A vector (see F.R. Blattner et al., Science, 196 161, 1977) to obtain hybrid DNA containing the DNA of human origin between the right arm and left arm of the Charon 4A vector. The ligation was carried out in a reaction mixture containing 66 mM Tris-HCl (pH 7.6), 6.6 mM MgCl₂ , 10 mM dithiothreitol and 1 mM ATP, at 11°C for 12 hours using T4 DNA ligase (Bethesda Research Laboratories). The hybrid DNA was subjected to in vitro packaging (A. Becker, M. Gold, Proc. Natl. Acad. Sci. U.S.A. 72, 581 (1975) using an Amersham kit to obtain $1.8 \times 10^6$ PFU/μg of a human gene library containing at least 99% of human DNA.

Reference Example 3. Screening of gene for human antibody H chain gene

A Charon 4A phage library containing human DNA prepared in Reference Example 2, was infected to Echerichia coli LE392 (ATCC 33572) to form plaques. Clones containing the human antibody H chain gene were selected using a 32P-labeled human antibody H chain J gene according to a plaque hybridization method (see, W.D. Benton, R.W. Davis, Science, Vol 196, 180, 1977). From the selected Charon 4A phage, a phage DNA containing a gene for the human antibody H chain gene was isolated according to the Thomas and Davis Method (M. Thomas and R.W. Davis, J. Mol. Biol., 91, 315, 1974).

Reference Example 4. Preparation of restriction enzyme cleavage map of human H chain gene

1 μg of the phage DNA containing the human gene obtained in Reference Example 3 was dissolved in 20 μl of a restriction enzyme cleavage buffer, and digested with 2 units of a restriction enzyme at 37°C for at least one hour.

When BstE II was used, the cleavage was carried out at 60°C for at least one hour.

The restriction enzyme cleavage buffer was an aqueous buffer solution containing 50 mM Tris-HCl (pH 7.4), 100 mM NaCl, and 10 mM MgSO₄ for EcoR I, EcoR V, Mlu I or Sph I; an aqueous buffer solution containing 10 mM Tris-HCl (pH 7.5), 60 mM NaCl, 7 mM MgCl₂ , 10 mM MgSO₄ , and 1 mM dithiothreitol for Aat II, Ava I, BamH I, BstE II, Hinc II, Hind III, Pst I or Pvu II; or an aqueous buffer solution containing 10 mM Tris-HCl (pH 8.0), 20 mM KCl, 7 mM MgCl₂ , and 7 mM 2-mercaptoethanol for Hpa I.

When two restriction endonucleases were used to digest DNA, the DNA was first treated with a restriction endonuclease effective at a low salt concentration, and after the salt concentration was increased to a predetermined high concentration, treated with another restriction endonuclease reactive at the high salt concentration.

After cleavage with a restriction enzyme, 4 μl of an aqueous solution containing 0.25% bromophenol blue and 50% glycerol was added to the cleavage product, which was then subjected to agarose gel electrophoresis (agarose concentration 0.8 to 2.5%). The agarose was the type II for electrophoresis obtained from Sigma. The electrophoresis buffer was a buffer containing 40 mM Tris-acetate (pH 8.0), and 1 mM EDTA. Electrophoresis was carried out using a vertical gel having a thickness of 2 mm at a voltage of 6 to 9 V/cm for 1.5 to 3 hours. In this electrophoresis, the molecular weight standard was based on a digestion product of the λ phage DNA with restriction enzyme Hind III (Nippon Gene). After the electrophoresis, DNA bands in the agarose gel were stained with a 2 μg/ml ethidium bromide aqueous solution, and radiated with long wavelength ultraviolet light to determine the cleavage pattern. The cleavage patterns obtained by cleavage with a single restriction enzyme separately using various restriction enzymes, and by cleavage with various combinations of two restriction enzymes, were analyzed to determine the relationship between the various restriction enzyme cleavage sites.

Reference Example 5. Recloning of human antibody H chain gene (construction of hybrid DNA containing Hind III fragment of 8.2 Kb containing human Cγ1 gene and E. coli plasmid pBR322)

3 μg of the Charon 4A phage DNA containing the human antibody gene was cleaved with Hind III according to the same procedure as described in Reference Example 4, and the cleavage product was subjected to agarose gel electrophoresis. An agarose gel portion containing a band corresponding to a DNA fragment of about 8.2 Kb containing the Cγ1 gene was cut off, and the agarose section was dissolved in a three-fold (vol/wt) amount of an 8M NaClO₃ aqueous solution. A DNA fragment of about 8.2 Kb was recovered from the aqueous section by a Chen and Thomas glass filter method (C.W. Chen and C.A. Thomas, Jr. Anal. Biochem. 101, 399, 1980).

On the other hand, 1 μg of an E. coli plasmid pBR322 was cleaved with Hind III according to the same procedure as described in Example 4, 0.5 unit of alkaline phosphatase (E. coli C75; Takara Shuzo) was added to the reaction mixture, and reaction was carried out at 68°C for one hour. After the reaction, the reaction mixture was extracted three times with phenol to inactivate and eliminate alkaline phosphatase. The Hind III/alkaline phosphatase-treated pBR322 solution thus obtained was mixed with an 8.2 Kb Hind III fragment aqueous solution recovered from gel as described above, and after ethanol precipitation, the precipitate was dissolved in a ligation buffer (see, Reference Example 2). To the solution was added 2 units of T4 DNA ligase, and reaction was carried out at 11°C for 12 hours to obtain a hybrid DNA.

The thus-obtained hybrid DNA was used to transform E. coliC600 (ATCC 33525) according to a modified CaCl₂ method (see, M.V. Norgard, K. Keen, J. Monaham, Gene, 3, 279, 1978). That is, an 18-hour culture of E. coli C600 was inoculated into 5 ml of L medium (1% trypton, 0.5% yeast extract 0.5% NaCl, pH 7.5) and was grown until the optical density at 600 nm reached 0.3. Cells were washed twice in a cold magnesium buffer (0.1 M NaCl, 5 mM MgCl₂, 5 mM Tris-HCl, pH 7.6, 0°C), the washed cells were resuspended in 2 ml of a cold calcium buffer (100 mM CaCl₂, 250 mM KCl, 5 mM MgCl₂, 5 mM Tris-HCl, pH 7.6, 0°C), and the suspension was allowed to stand at 0°C for 25 minutes. The cells were then collected, resuspended in a calcium buffer at a 10-fold concentration in relation to the original concentration, and the concentrated suspension was mixed with the above-mentioned hybrid DNA aqueous solution at a volume ratio of 2:1. The mixture was maintained at 0°C for 60 minutes, 1 ml of LBG medium (1% trypton, 0.5% yeast extract, 1% NaCl, and 0.08% glucose, pH 7.5) were added to the mixture, and culturing was carried out at 37°C for 1 hour. The cultured broth was plated on a selective medium (L medium plate supplemented with 30 μg/ml ampicillin) at a concentration of 100 μl/plate. The plate was incubated at 37°C overnight to allow a growth of transformants. The developed colonies were treated by a known method to prepare DNA, and the target hybrid DNA was confirmed by agarose gel electrophoresis.

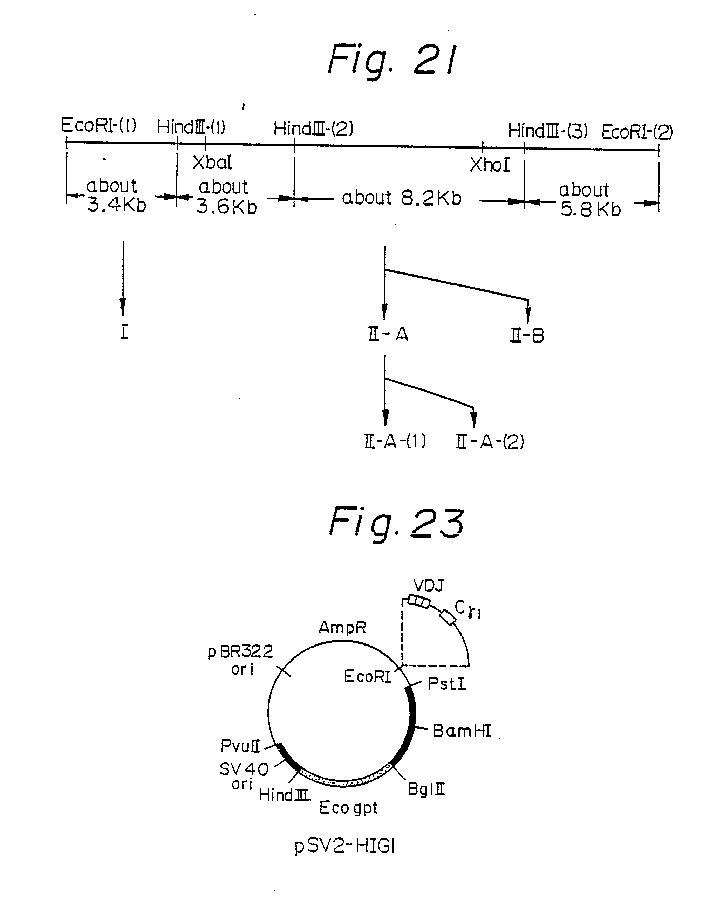

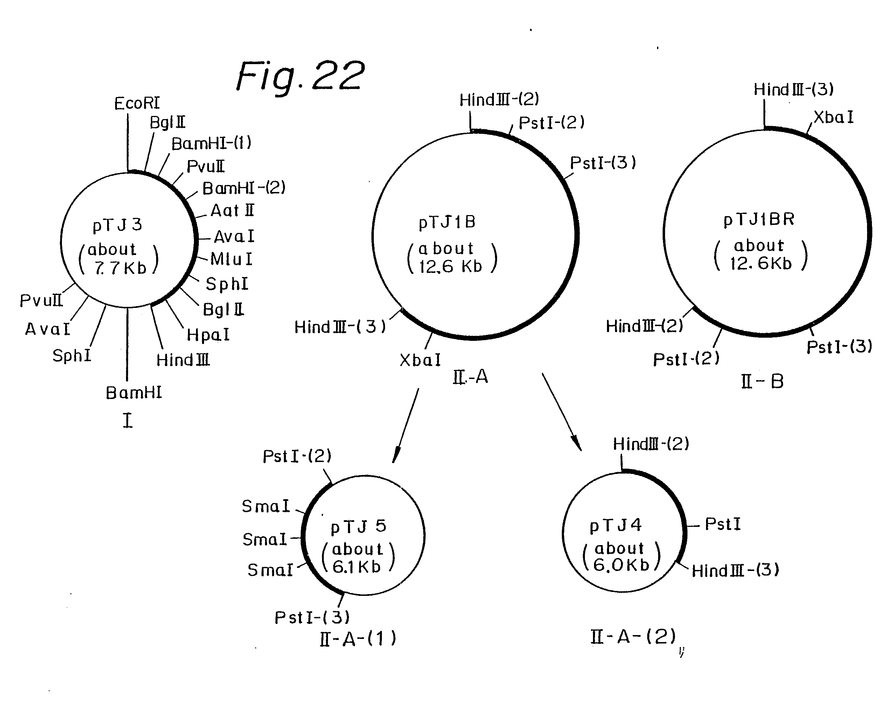

The restriction endonuclease cleavage maps thus obtained are shown in Figs. 21 and 22. Figure 21 represents the map of DNA obtained by cleaving a chromosomal DNA containing the human antibody gene with a restriction endonuclease EcoR I. In this map, a 5′ end is shown as EcoR I-(1); and a 3′ end is shown as EcoR I(2). Figure 21 also shows the cleavage sites of restriction endonuclease Xba I and Xho I.

This DNA fragment was cleaved with appropriate restriction endonucleases to separate the DNA fragment into small fragments. As shown in Fig. 22, some of the small fragments were linked to E. coli plasmid pBR322 to obtain four subclones.

## Endonuclease cleavage maps of subclones

### Subclone pTJ1B

A subclone pTJ1B was obtained by inserting the small DNA fragment of about 8.2 Kb flanked by Hind III(2) and Hind III(3) into Hind III site of pBR322. Restriction endonuclease cleavage map of this subclone is shown in Fig. 22 as II-A. There are three to four Pst I sites between Pst I(3) and Hind III(3) in II-A.

### Subclone pTJ1BR

This subclone is same as the above-mentioned subclone pTJ1B except that the DNA fragment is reversely inserted. A restriction endonuclease cleavage map of this subclone is shown in Fig. 22 as IIB.

### Subclone pTJ5

This subclone was obtained by inserting the small DNA fragment flanked by Pst I(2) and Pst I-(3) into the Pst I site of pBR322. A restriction endonuclease cleavage map of this subclone is shown in Fig. 22 as IIA-(1).

### Subclone pTJ4

This subclone was obtained by deleting a DNA fragment starting from a Pst I site present between Hind III(3) and Pst I(3) and adjacent to the Hind III-(3) and ending with Pst I(2), from the above-mentioned subclone pTJ1B. A restriction endonuclease cleavage map of this subclone is shown in Fig. 22 as IIA-(2).

### Subclone pTJ3

This subclone was obtained by inserting the small DNA fragment of about 3.4 Kb flanked by EcoR I(1) and Hind III(1) between the EcoR I site and Hind III site of pBR322. A restriction endonuclease cleavage map of this clone is shown in Fig. 22 as I.

## Reference Example 6. Determination of nucleotide sequence of human antibody H chain gene enhancer region

A nucleotide sequence of the enhancer region of the human antibody H chain gene was determined by the Maxam-Gilbert method (A. Maxam and W. Gilbert, Methods Enzymal. 65, 499, 1980).

About 50 $\mu$g of the subclone pTJ3 prepared according to the procedure described in Reference Example 4 was cleaved with BstE II. The resulting DNA fragment was dephesphorylated with alkaline phosphatase, and labeled with [$\gamma$-$^{32}$P]ATP using 5 units of polynucleotide kinase (P.L.-Biochemicals). The $^{32}$P-labeled DNA fragments was digested with Hpa I, and a target DNA fragment was separated by polyacrylamide gel electrophoresis (gel concentration 5%) and extracted from the gel. The resulting $^{32}$P-labeled BstE II/Hpa I fragment was subjected to partial digestion, and the digested products were separated by electrophoresis on polyacrylamide gel electrophoresis (gel concentration 8 to 23%) in 7M urea. Autoradiography was carried out at -80°C for 2 to 7 days, and the degradation pattern was analyzed to determine the nucleotide sequence of the enhancer region. The nucleotide sequence is shown in Fig. 5.

## Reference Example 7. Construction of plasmid for expression of human antibody gene

EcoR I fragment (HIG1) of about 21 Kb containing a human antibody H chain gene was inserted into the EcoR I site of pSV2-gpt (R.C. Mulligan and P. Berg, Proc. Natl. Acad. Sci. USA, 78, 2072 (1981) to form pSV2-HIG1. A restriction endonuclease cleavage map of this plasmid is shown in Fig. 23.

## Claims

1. A mouse-human chimera antibody reactive with a human common acute lymphocytic leukemia antigen, comprising an H chain comprising a V region of mouse origin and a C region of human origin; and an L chain comprising a V region of mouse origin and a C region of human origin.

2. An antibody according to claim 1, wherein the V regions of mouse origin are those of an antibody reactive with a human common acute lymphocytic leukemia antigen.

3. An antibody according to claim 1, wherein the C region of the L chain is derived from a x chain of a human antibody, and the C region of the H chain is derived from a C$_\gamma$1 chain of a human antibody.

4. An antibody according to claim 1, wherein the V region of the H chain has the following amino acid sequence:

Asp Val Gln Leu Val Glu Ser Gly Gly Gly
Leu Val Gln Pro Gly Gly Ser Arg Lys Leu Ser Cys Ala Ala
Ser Gly Phe Thr Phe Ser Ser Phe Gly Met His Trp Val Arg
Gln Ala Pro Glu Lys Gly Leu Glu Trp Val Ala Tyr Ile Ser
Gly Gly Ser Tyr Thr Ile Tyr Tyr Ala Asp Thr Val Lys Gly
Arg Phe Thr Ile Ser Arg Asp Asn Pro Lys Asn Thr Leu Phe
Leu Gln Met Thr Ser Leu Arg Ser Glu Asp Thr Ala Met Tyr
Tyr Cys Ala Ser Ser Tyr Gly Asn Phe Trp Tyr Phe Asp Val
Trp Gly Ala Gly Thr Thr Val Thr Val Ser Ser.

5. An antibody according to claim 1, wherein the C region of the H chain has the following amino acid sequence:

Ala Ser Thr Lys Gly Pro Ser Val Phe Pro
Leu Ala Pro Ser Ser Lys Ser Thr Ser Gly Gly Thr Ala Ala
Leu Gly Cys Leu Val Lys Asp Tyr Phe Pro Glu Pro Val Thr
Val Ser Trp Asn Ser Gly Ala Leu Thr Ser Gly Val His Thr
Phe Pro Ala Val Leu Gln Ser Ser Gly Leu Tyr Ser Leu Ser
Ser Val Val Thr Val Pro Ser Ser Ser Leu Gly Thr Gln Thr
Tyr Ile Cys Asn Val Asn His Lys Pro Ser Asn Thr Lys Val
Asp Lys Lys Val Glu Pro Lys Ser Cys Asp Lys Thr His Thr
Cys Pro Pro Cys Pro Ala Pro Glu Leu Leu Gly Gly Pro Ser
Val Phe Leu Phe Pro Pro Lys Pro Lys Asp Thr Leu Met Ile
Ser Arg Thr Pro Glu Val Thr Cys Val Val Val Asp Val Ser
His Glu Asp Pro Glu Val Lys Phe Asn Trp Tyr Val Asp Gly
Val Glu Val His Asn Ala Lys Thr Lys Pro Arg Glu Glu Gln
Tyr Asn Ser Thr Tyr Arg Val Val Ser Val Leu Thr Val Leu
His Gln Asp Trp Leu Asn Gly Lys Glu Tyr Lys Cys Lys Val
Ser Asn Lys Ala Leu Pro Ala Pro Ile Glu Lys Thr Ile Ser

Lys Ala Lys Gly Gln Pro Arg Glu Pro Gln Val Tyr Thr Leu
Pro Pro Ser Arg Glu Glu Met Thr Lys Asn Gln Val Ser Leu
Thr Cys Leu Val Lys Gly Phe Tyr Pro Ser Asp Ile Ala Val
Glu Trp Glu Ser Asn Gly Gln Pro Glu Asn Asn Tyr Lys Thr
Thr Pro Pro Val Leu Asp Ser Asp Gly Ser Phe Phe Leu Tyr
Ser Lys Leu Thr Val Asp Lys Ser Arg Trp Gln Gln Gly Asn
Val Phe Ser Cys Ser Val Met His Glu Ala Leu His Asn His
Tyr Thr Gln Lys Ser Leu Ser Leu Ser Pro Gly Lys.

6. An antibody according to claim 1, wherein the V region of the L chain has the following amino acid sequence:

Glu Ile Val Leu Thr Gln Ser Pro Ala Leu
Met Ala Ala Ser Pro Gly Gly Lys Val Thr Ile Arg Cys Ser
Val Ser Ser Ser Ile Ser Ser Ser Asn Leu His Trp Tyr Gln
Gln Lys Ser Glu Thr Ser Pro Lys Pro Trp Ile Tyr Gly Thr
Ser Asn Leu Ala Ser Gly Val Pro Val Arg Phe Ser Gly Ser
Gly Ser Gly Thr Ser Tyr Phe Leu Thr Ile Ser Ser Met Glu
Ala Glu Asp Ala Ala Thr Tyr Tyr Cys Gln Gln Trp Ser Ser
Tyr Pro Leu.

7. An antibody according to claim 1, wherein the C region of the L chain has the following amino acid sequence:

Thr Val Ala Ala Pro Ser Val Phe Ile Phe
Pro Pro Ser Asp Glu Gln Leu Lys Ser Gly Thr Ala Ser Val
Val Cys Leu Leu Asn Asn Phe Tyr Pro Arg Glu Ala Lys Val
Gln Trp Lys Val Asp Asn Ala Leu Gln Ser Gly Asn Ser Gln
Glu Ser Val Thr Glu Gln Asp Ser Lys Asp Ser Thr Tyr Ser
Leu Ser Ser Thr Leu Thr Leu Ser Lys Ala Asp Tyr Glu Lys
His Lys Val Tyr Ala Cys Glu Val Thr His Gln Gly Leu Ser
Ser Pro Val Thr Lys Ser Phe Asn Arg Gly Glu Cys.

8. A mouse-human chimera antibody L chain comprising a V region of a mouse antibody reactive with a human common acute lymphocytic leukemia antigen and a C region of human origin.

9. An L chain according to claim 8, wherein the V region has at least the following amino acid sequence:

Glu Ile Val Leu Thr Gln Ser Pro Ala Leu
Met Ala Ala Ser Pro Gly Glu Lys Val Thr Ile Arg Cys Ser

Val Ser Ser Ser Ile Ser Ser Ser Asn Leu His Trp Tyr Gln

Gln Lys Ser Glu Thr Ser Pro Lys Pro Trp Ile Tyr Gly Thr

Ser Asn Leu Ala Ser Gly Val Pro Val Arg Phe Ser Gly Ser

Gly Ser Gly Thr Ser Tyr Phe Leu Thr Ile Ser Ser Met Glu

Ala Glu Asp Ala Ala Thr Tyr Tyr Cys Gln Gln Trp Ser Ser

Tyr Pro Leu.

10. An L chain according to claim 8, wherein the C region has at least the following amino acid sequence:

Thr Val Ala Ala Pro Ser Val Phe Ile Phe

Pro Pro Ser Asp Glu Gln Leu Lys Ser Gly Thr Ala Ser Val

Val Cys Leu Leu Asn Asn Phe Tyr Pro Arg Glu Ala Lys Val

Gln Trp Lys Val Asp Asn Ala Leu Gln Ser Gly Asn Ser Gln

Glu Ser Val Thr Glu Gln Asp Ser Lys Asp Ser Thr Tyr Ser

Leu Ser Ser Thr Leu Thr Leu Ser Lys Ala Asp Tyr Glu Lys

His Lys Val Tyr Ala Cys Glu Val Thr His Gln Gly Leu Ser

Ser Pro Val Thr Lys Ser Phe Asn Arg Gly Glu Cys.

11. A V region of an L chain of a mouse antibody reactive with a human common acute lymphocytic leukemia antigen.

12. A V region according to claim 11, wherein the V region has at least the following amino acid sequence:

Glu Ile Val Leu Thr Gln Ser Pro Ala Leu

Met Ala Ala Ser Pro Gly Glu Lys Val Thr Ile Arg Cys Ser

Val Ser Ser Ser Ile Ser Ser Ser Asn Leu His Trp Tyr Gln

Gln Lys Ser Glu Thr Ser Pro Lys Pro Trp Ile Tyr Gly Thr

Ser Asn Leu Ala Ser Gly Val Pro Val Arg Phe Ser Gly Ser

Gly Ser Gly Thr Ser Tyr Phe Leu Thr Ile Ser Ser Met Glu

Ala Glu Asp Ala Ala Thr Tyr Tyr Cys Gln Gln Trp Ser Ser

Tyr Pro Leu.

13. A mouse-human chimera antibody H chain comprising a V region of a mouse antibody reactive with a human common acute lymphocytic leukemia antigen and a C region of human origin.

14. An H chain according to claim 13, wherein the V region has at least the following amino acid sequence:

Asp Val Gln Leu Val Glu Ser Gly Gly Gly

Leu Val Gln Pro Gly Gly Ser Arg Lys Leu Ser Cys Ala Ala

Ser Gly Phe Thr Phe Ser Ser Phe Gly Met His Trp Val Arg

Gln Ala Pro Glu Lys Gly Leu Glu Trp Val Ala Tyr Ile Ser

Gly Gly Ser Tyr Thr Ile Tyr Tyr Ala Asp Thr Val Lys Gly

Arg Phe Thr Ile Ser Arg Asp Asn Pro Lys Asn Thr Leu Phe

Leu Gln Met Thr Ser Leu Arg Ser Glu Asp Thr Ala Met Tyr

Tyr Cys Ala Ser Ser Tyr Gly Asn Phe Trp Tyr Phe Asp Val

Trp Gly Ala Gly Thr Thr Val Thr Val Ser Ser.

15. An H chain according to claim 13, wherein the C region has at least the following amino acid sequence:

Ala Ser Thr Lys Gly Pro Ser Val Phe Pro

Leu Ala Pro Ser Ser Lys Ser Thr Ser Gly Gly Thr Ala Ala

Leu Gly Cys Leu Val Lys Asp Tyr Phe Pro Glu Pro Val Thr

Val Ser Trp Asn Ser Gly Ala Leu Thr Ser Gly Val His Thr

Phe Pro Ala Val Leu Gln Ser Ser Gly Leu Tyr Ser Leu Ser

Ser Val Val Thr Val Pro Ser Ser Ser Leu Gly Thr Gln Thr

Tyr Ile Cys Asn Val Asn His Lys Pro Ser Asn Thr Lys Val

Asp Lys Lys Val Glu Pro Lys Ser Cys Asp Lys Thr His Thr

Cys Pro Pro Cys Pro Ala Pro Glu Leu Leu Gly Gly Pro Ser

Val Phe Leu Phe Pro Pro Lys Pro Lys Asp Thr Leu Met Ile

Ser Arg Thr Pro Glu Val Thr Cys Val Val Val Asp Val Ser

His Glu Asp Pro Glu Val Lys Phe Asn Trp Tyr Val Asp Gly

Val Glu Val His Asn Ala Lys Thr Lys Pro Arg Glu Glu Gln

Tyr Asn Ser Thr Tyr Arg Val Val Ser Val Leu Thr Val Leu

His Gln Asp Trp Leu Asn Gly Lys Glu Tyr Lys Cys Lys Val

Ser Asn Lys Ala Leu Pro Ala Pro Ile Glu Lys Thr Ile Ser

Lys Ala Lys Gly Gln Pro Arg Glu Pro Gln Val Tyr Thr Leu

Pro Pro Ser Arg Glu Glu Met Thr Lys Asn Gln Val Ser Leu

Thr Cys Leu Val Lys Gly Phe Tyr Pro Ser Asp Ile Ala Val

Glu Trp Glu Ser Asn Gly Gln Pro Glu Asn Asn Tyr Lys Thr

Thr Pro Pro Val Leu Asp Ser Asp Gly Ser Phe Phe Leu Tyr

Ser Lys Leu Thr Val Asp Lys Ser Arg Trp Gln Gln
Gly Asn

Val Phe Ser Cys Ser Val Met His Glu Ala Leu His
Asn His

Tyr Thr Gln Lys Ser Leu Ser Leu Ser Pro Gly Lys.

16. A V region of an H chain of a mouse antibody reactive with a human common acute lymphocytic leukemia antigen.

17. A V region according to claim 16, wherein the V region has at least the following amino acid sequence:

Asp Val Gln Leu Val Glu Ser Gly Gly Gly

Leu Val Gln Pro Gly Gly Ser Arg Lys Leu Ser Cys
Ala Ala

Ser Gly Phe Thr Phe Ser Ser Phe Gly Met His Trp
Val Arg

Gln Ala Pro Glu Lys Gly Leu Glu Trp Val Ala Tyr
Ile Ser

Gly Gly Ser Tyr Thr Ile Tyr Tyr Ala Asp Thr Val
Lys Gly

Arg Phe Thr Ile Ser Arg Asp Asn Pro Lys Asn Thr
Leu Phe

Leu Gln Met Thr Ser Leu Arg Ser Glu Asp Thr Ala
Met Tyr

Tyr Cys Ala Ser Ser Tyr Gly Asn Phe Trp Tyr Phe
Asp Val

Trp Gly Ala Gly Thr Thr Val Thr Val Ser Ser.

18. A nucleotide sequence for expression of a mouse-human chimera L chain wherein a V region of the L chain is that of a mouse antibody reactive with a human common acute lymphocytic leukemia antigen, comprising:
a DNA fragment coding for a V region of the L chain of mouse origin;
a DNA fragment coding for a C region of the L chain of human origin; and
an enhancer DNA sequence of a human H chain gene origin.

19. A nucleotide sequence according to claim 18, wherein the DNA fragment coding for V region codes for at least the following amino acid sequence:

Glu Ile Val Leu Thr Gln Ser Pro Ala Leu

Met Ala Ala Ser Pro Gly Glu Lys Val Thr Ile Arg
Cys Ser

Val Ser Ser Ser Ile Ser Ser Ser Asn Leu His Trp
Tyr Gln

Gln Lys Ser Glu Thr Ser Pro Lys Pro Trp Ile Tyr
Gly Thr

Ser Asn Leu Ala Ser Gly Val Pro Val Arg Phe Ser
Gly Ser

Gly Ser Gly Thr Ser Tyr Phe Leu Thr Ile Ser Ser
Met Glu

Ala Glu Asp Ala Ala Thr Tyr Tyr Cys Gln Gln Tyr
Ser Ser

Tyr Pro Leu.

20. A nucleotide sequence according to claim 18, wherein the DNA fragment coding for the C region codes for at least the following amino acid sequence:

Thr Val Ala Ala Pro Ser Val Phe Ile Phe

Pro Pro Ser Asp Glu Gln Leu Lys Ser Gly Thr Ala
Ser Val

Val Cys Leu Leu Asn Asn Phe Tyr Pro Arg Glu Ala
Lys Val

Gln Trp Lys Val Asp Asn Ala Leu Gln Ser Gly Asn
Ser Gln

Glu Ser Val Thr Glu Gln Asp Ser Lys Asp Ser Thr
Tyr Ser

Leu Ser Ser Thr Leu Thr Leu Ser Lys Ala Asp Tyr
Glu Lys

His Lys Val Tyr Ala Cys Glu Val Thr His Gln Gly
Leu Ser

Ser Pro Val Thr Lys Ser Phe Asn Arg Gly Glu Cys.

21. A nucleotide sequence according to claim 18, wherein the DNA fragment coding for the V region has at least the following nucleotide sequence:

GA AATTGTGCTC ACCCAGTCTC CAGCACTCAT
GGCTGCTTCT CCAGGGGAGA AGGTCACCAT
CCGCTGCAGT GTCAGCTCAA
GTATAAGTTC CAGCAACTTG CACTGGTACC AGCAGAAGTC AGAAACCTCC
CCCAAACCCT GGATTTATGG CACATCCAAC
CTGGCTTCTG GAGTCCCTGT
TCGTTTCAGT GGCAGTGGAT CTGGGACCTC
TTATTTTCTC ACAATCAGCA
GCATGGAGGC TGAAGATGCT GCCACTTATT
ACTGTCAAGA GTGGAGTAGT
TACCCACTC.

22. A nucleotide sequence according to claim 18, wherein the DNA fragment coding for the C region has the following nucleotide sequence:
ACTGTGGCTG CACCATCTGT CTTCATCTTC
CCGCCATCTG
ATGAGCAGTT GAAATCTGGA ACTGCCTCTG
TTGTGTGCCT GCTGAATAAC
TTCTATCCCA GAGAGGCCAA AGTACAGTGG
AAGGTGGATA ACGCCCTCCA
ATCGGGTAAC TCCCAGGAGA GTGTCACAGA
GCAGGACAGC AAGGACAGCA
CCTACAGCCT CAGCAGCACC CTGACGCTGA
GCAAAGCAGA CTACGAGAAA
CACAAAGTCT ACGCCTGCGA AGTCACCCAT
CAGGGCCTGA GCTCGCCCGT
CACAAAGAGC TTCAACAGGG GAGAGTGT.

23. A nucleotide sequence according to claim 18, wherein the enhancer DNA sequence is the following sequence:
TTGGCGAGCTGGAAGCAGATGATGAATTAG
AGTCAAGATGGCTGCATGGGGGTCTCCGGC
ACCCACAGCAGGTGGCAGGAAGCAGGTCAC
CGCGAGAGTCTATTTTAGGAAGCAAAAAAA
CACAATTGGTAAATTTATCACTTCTGGTTG

TGAAGAGGTGGTTTTGCCAGGCCCAGATCT
GAAAGTGCTCTACTGAGCAAAACAACACTT
GGACAATTTGCGTTTCTAAAATAAGGCGAG
GCTGACCGAAATCGAAAGGCTTTTTTTAAC
TATCTGCAATTTCATTTCCAATCTTAGCTT
ATCAACTGCTAGTTGG

24. A nucleotide sequence according to claim 18, wherein the nucleotide sequence contains the enhancer DNA sequence, the DNA fragment coding for the V region and the DNA fragment coding for the C region, in this order.

25. A nucleotide sequence for expression of a chimera mouse-human H chain wherein the V region of the H chain is that of a mouse antibody reactive with a human common acute lymphocytic leukemia antigen, comprising:

a DNA fragment coding for a V region of the H chain of mouse origin;

a DNA fragment coding for a C region of the H chain of human origin; and

an enhancer DNA sequence of a human H chain gene origin; and optionally,

an enhancer DNA sequence of a mouse H chain gene origin.

26. A nucleotide sequence according to claim 25, wherein the DNA fragment coding for the V region codes for at least the following amino acid sequence:

Asp Val Gln Leu Val Glu Ser Gly Gly Gly

Leu Val Gln Pro Gly Gly Ser Arg Lys Leu Ser Cys Ala Ala

Ser Gly Phe Thr Phe Ser Ser Phe Gly Met His Trp Val Arg

Gln Ala Pro Glu Lys Gly Leu Glu Trp Val Ala Tyr Ile Ser

Gly Gly Ser Tyr Thr Ile Tyr Tyr Ala Asp Thr Val Lys Gly

Arg Phe Thr Ile Ser Arg Asp Asn Pro Lys Asn Thr Leu Phe

Leu Gln Met Thr Ser Leu Arg Ser Glu Asp Thr Ala Met Tyr

Tyr Cys Ala Ser Ser Tyr Gly Asn Phe Trp Tyr Phe Asp Val

Trp Gly Ala Gly Thr Thr Val Thr Val Ser Ser.

27. A nucleotide sequence according to claim 25, wherien the DNA fragment coding for the C region codes for at least the following amino acid sequence:

Ala Ser Thr Lys Gly Pro Ser Val Phe Pro

Leu Ala Pro Ser Ser Lys Ser Thr Ser Gly Gly Thr Ala Ala

Leu Gly Cys Leu Val Lys Asp Tyr Phe Pro Glu Pro Val Thr

Val Ser Trp Asn Ser Gly Ala Leu Thr Ser Gly Val His Thr

Phe Pro Ala Val Leu Gln Ser Ser Gly Leu Tyr Ser Leu Ser

Ser Val Val Thr Val Pro Ser Ser Ser Leu Gly Thr Gln Thr

Tyr Ile Cys Asn Val Asn His Lys Pro Ser Asn Thr Lys Val

Asp Lys Lys Val Glu Pro Lys Ser Cys Asp Lys Thr His Thr

Cys Pro Pro Cys Pro Ala Pro Glu Leu Leu Gly Gly Pro Ser

Val Phe Leu Phe Pro Pro Lys Pro Lys Asp Thr Leu Met Ile

Ser Arg Thr Pro Glu Val Thr Cys Val Val Val Asp Val Ser

His Glu Asp Pro Glu Val Lys Phe Asn Trp Tyr Val Asp Gly

Val Glu Val His Asn Ala Lys Thr Lys Pro Arg Glu Glu Gln

Tyr Asn Ser Thr Tyr Arg Val Val Ser Val Leu Thr Val Leu

His Gln Asp Trp Leu Asn Gly Lys Glu Tyr Lys Cys Lys Val

Ser Asn Lys Ala Leu Pro Ala Pro Ile Glu Lys Thr Ile Ser

Lys Ala Lys Gly Gln Pro Arg Glu Pro Gln Val Tyr Thr Leu

Pro Pro Ser Arg Glu Glu Met Thr Lys Asn Gln Val Ser Leu

Thr Cys Leu Val Lys Gly Phe Tyr Pro Ser Asp Ile Ala Val

Glu Trp Glu Ser Asn Gly Gln Pro Glu Asn Asn Tyr Lys Thr

Thr Pro Pro Val Leu Asp Ser Asp Gly Ser Phe Phe Leu Tyr

Ser Lys Leu Thr Val Asp Lys Ser Arg Trp Gln Gln Gly Asn

Val Phe Ser Cys Ser Val Met His Glu Ala Leu His Asn His

Tyr Thr Gln Lys Ser Leu Ser Leu Ser Pro Gly Lys.

28. A nucleotide sequence coding for a V region of the L chain of a mouse antibody reactive with a human common acute lymphocytic leukemia antigen.

29. A nucleotide sequence according to claim 28, wherein the nucleotide sequence codes for at least the following amino acid sequence:

Glu Ile Val Leu Thr Gln Ser Pro Ala Leu

Met Ala Ala Ser Pro Gly Glu Lys Val Thr Ile Arg Cys Ser

Val Ser Ser Ser Ile Ser Ser Ser Asn Leu His Trp Tyr Gln

Gln Lys Ser Glu Thr Ser Pro Lys Pro Trp Ile Tyr Gly Thr

Ser Asn Leu Ala Ser Gly Val Pro Val Arg Phe Ser Gly Ser

Gly Ser Gly Thr Ser Tyr Phe Leu Thr Ile Ser Ser Met Glu

Ala Glu Asp Ala Ala Thr Tyr Tyr Cys Gln Gln Trp Ser Ser

Tyr Pro Leu.

30. A nucleotide sequence according to claim 28, wherein the nucleotide sequence has at least the following nucleotide sequence:

GA AATTGTGCTC ACCCAGTCTC CAGCACTCAT GGCTGCTTCT CCAGGGGAGA AGGTCACCAT CCGCTGCAGT GTCAGCTCAA GTATAAGTTC CAGCAACTTG CACTGGTACC AGCAGAAGTC AGAAACCTCC CCCAAACCCT GGATTTATGG CACATCCAAC CTGGCTTCTG GAGTCCCTGT TCGTTTCAGT GGCAGTGGAT CTGGGACCTC TTATTTTCTC ACAATCAGCA GCATGGAGGC TGAAGATGCT GCCACTTATT ACTGTCAACA GTGGAGTAGT TACCCACTC.

31. A plasmid containing the nucleotide sequence of claim 18.

32. A plasmid containing the nucleotide sequence of claim 25.

33. An animal cell transformed with a plasmid of claim 32, capable of producing a chimera H chain.

34. An animal cell transformed with a plasmid of claim 31 and a plasmid of claim 32, capable of producing a chimera antibody reactive with a human common acute lymphocytic leukemia antigen.

35. A process for production of the chimera antibody comprising culturing the animal cells of claim 34, and recovering the chimera antibody.

# Fig. 1

```
                                        10
Asp Val Gln Leu Val Glu Ser Gly Gly Gly

                                        20
Leu Val Gln Pro Gly Gly Ser Arg Lys Leu

                                        30
Ser Cys Ala Ala Ser Gly Phe Thr Phe Ser

                                        40
Ser Phe Gly Met His Trp Val Arg Gln Ala

                                        50
Pro Glu Lys Gly Leu Glu Trp Val Ala Tyr

                                        60
Ile Ser Gly Gly Ser Tyr Thr Ile Tyr Tyr

                                        70
Ala Asp Thr Val Lys Gly Arg Phe Thr Ile

                                        80
Ser Arg Asp Asn Pro Lys Asn Thr Leu Phe

                                        90
Leu Gln Met Thr Ser Leu Arg Ser Glu Asp

                                       100
Thr Ala Met Tyr Tyr Cys Ala Ser Ser Tyr

                                       110
Gly Asn Phe Trp Tyr Phe Asp Val Trp Gly Ala Gly Thr Thr Val Thr Val Ser Ser
```

# Fig. 2-1

```
                                                   10
Ala Ser Thr Lys Gly Pro Ser Val Phe Pro

                                                   20
Leu Ala Pro Ser Ser Lys Ser Thr Ser Gly

                                                   30
Gly Thr Ala Ala Leu Gly Cys Leu Val Lys

                                                   40
Asp Tyr Phe Pro Glu Pro Val Thr Val Ser

                                                   50
Trp Asn Ser Gly Ala Leu Thr Ser Gly Val

                                                   60
His Thr Phe Pro Ala Val Leu Gln Ser Ser

                                                   70
Gly Leu Tyr Ser Leu Ser Ser Val Val Thr

                                                   80
Val Pro Ser Ser Ser Leu Gly Thr Gln Thr

                                                   90
Tyr Ile Cys Asn Val Asn His Lys Pro Ser

                                                   100
Asn Thr Lys Val Asp Lys Lys Val Glu Pro

                                                   110
Lys Ser Cys Asp Lys Thr His Thr Cys Pro

                                                   120
Pro Cys Pro Ala Pro Glu Leu Leu Gly Gly

                                                   130
Pro Ser Val Phe Leu Phe Pro Pro Lys Pro

                                                   140
Lys Asp Thr Leu Met Ile Ser Arg Thr Pro

                                                   150
Glu Val Thr Cys Val Val Val Asp Val Ser
```

# Fig. 2-2

```
                                             160
His Glu Asp Pro Glu Val Lys Phe Asn Trp

                                             170
Tyr Val Asp Gly Val Glu Val His Asn Ala

                                             180
Lys Thr Lys Pro Arg Glu Glu Gln Tyr Asn

                                             190
Ser Thr Tyr Arg Val Val Ser Val Leu Thr

                                             200
Val Leu His Gln Asp Trp Leu Asn Gly Lys

                                             210
Glu Tyr Lys Cys Lys Val Ser Asn Lys Ala

                                             220
Leu Pro Ala Pro Ile Glu Lys Thr Ile Ser

                                             230
Lys Ala Lys Gly Gln Pro Arg Glu Pro Gln

                                             240
Val Tyr Thr Leu Pro Pro Ser Arg Glu Glu

                                             250
Met Thr Lys Asn Gln Val Ser Leu Thr Cys

                                             260
Leu Val Lys Gly Phe Tyr Pro Ser Asp Ile

                                             270
Ala Val Glu Trp Glu Ser Asn Gly Gln Pro

                                             280
Glu Asn Asn Tyr Lys Thr Thr Pro Pro Val

                                             290
Leu Asp Ser Asp Gly Ser Phe Phe Leu Tyr

                                             300
Ser Lys Leu Thr Val Asp Lys Ser Art Trp
```

# Fig. 2-3

Gln Gln Gly Asn Val Phe Ser Cys Ser Val$^{310}$

Met His Glu Ala Leu His Asn His Tyr Thr$^{320}$

Gln Lys Ser Leu Ser Leu Ser Pro Gly Lys$^{330}$

# Fig. 3

```
                              .                      10
Glu Ile Val Leu Thr Gln Ser Pro Ala Leu

                                                     20
Met Ala Ala Ser Pro Gly Glu Lys Val Thr

                                                     30
Ile Arg Cys Ser Val Ser Ser Ser Ile Ser

                                                     40
Ser Ser Asn Leu His Trp Tyr Gln Gln Lys

                                                     50
Ser Glu Thr Ser Pro Lys Pro Trp Ile Tyr

                                                     60
Gly Thr Ser Asn Leu Ala Ser Gly Val Pro

                                                     70
Val Arg Phe Ser Gly Ser Gly Ser Gly Thr

                                                     80
Ser Tyr Phe Leu Thr Ile Ser Ser Met Glu

                                                     90
Ala Glu Asp Ala Ala Thr Tyr Tyr Cys Gln

                                                    100
Gln Trp Ser Ser Tyr Pro Leu Thr Phe Gly

Gly Gly Thr Lys Leu Glu Ile Lys Arg
```

# Fig. 4

```
                                          10
Thr Val Ala Ala Pro Ser Val Phe Ile Phe

                                          20
Pro Pro Ser Asp Glu Gln Leu Lys Ser Gly

                                          30
Thr Ala Ser Val Val Cys Leu Leu Asn Asn

                                          40
Phe Tyr Pro Arg Glu Ala Lys Val Gln Trp

                                          50
Lys Val Asp Asn Ala Leu Gln Ser Gly Asn

                                          60
Ser Gln Glu Ser Val Thr Glu Gln Asp Ser

                                          70
Lys Asp Ser Thr Tyr Ser Leu Ser Ser Thr

                                          80
Leu Thr Leu Ser Lys Ala Asp Tyr Glu Lys

                                          90
His Lys Val Tyr Ala Cys Glu Val Thr His

                                          100
Gln Gly Leu Ser Ser Pro Val Thr Lys Ser Phe Asn Arg Gly Glu Cys
```

# Fig. 5

TTGGCGAGCTGGAAGCAGATGATGAATTAG

AGTCAAGATGGCTGCATGGGGGTCTCCGGC

ACCCACAGCAGGTGGCAGGAAGCAGGTCAC

CGCGAGAGTCTATTTTAGGAAGCAAAAAAA

CACAATTGGTAAATTTATCACTTCTGGTTG

TGAAGAGGTGGTTTTGCCAGGCCCAGATCT

GAAAGTGCTCTACTGAGCAAAACAACACTT

GGACAATTTGCGTTTCTAAAATAAGGCGAG

GCTGACCGAAATCGAAAGGCTTTTTTTAAC

TATCTGCAATTTCATTTCCAATCTTAGCTT

ATCAACTGCTAGTTGG

# Fig. 6

```
GATGTGCAGCTGGTGGAGTCTGGGGGAGGCTTAGTGCAGCCT
|————————————————V-segment ——————————————
```

```
GGAGGGTCCCGGAAACTCTCCTGTGCAGCCTCTGGATTCACT
————————————————————————————————————————————
```

```
TTCAGTAGCTTTGGAATGCACTGGGTTCGTCAGGCTCCAGAG
————————————————————————————————————————————
```

```
AAGGGGCTGGAGTGGGTCGCATATATTAGTGGTGGCAGTTAT
————————————————————————————————————————————
```

```
ACCATCTACTATGCAGACACAGTGAAGGGCCGATTCACCATC
————————————————————————————————————————————
```

```
TCCAGAGACAATCCCAAGAACACCCTGTTCCTACAAATGACC
————————————————————————————————————————————
```

```
AGTCTAAGGTCTGAGGACACGGCCATGTATTACTGTGCAAGT
————————————————————————————————————————————
```

```
TCCTATGGTAACTTCTGGTACTTCGATGTCTGGGGCGCAGGG
————————————————————————————————————————————
```

```
ACCACGGTCACCGTCTCCTCATCCTATGGTAACTTCTGGTAC
————————————————————————————|—— D-segment ——|—|——————
```

```
TTCGATGTCTGGGGCGCAGGGACCACGGTCACCGTCTCCTCA
————————————————— J₁-segment ——————————————|
```

# Fig. 7-1

$C_H1$-segment;

GCCTCCACCAAGGGCCCATCGGTCTTCCCCCTGGCA

CCCTCCTCCAAGAGCACCTCTGGGGGCACAGCGGCC

CTGGGCTGCCTGGTCAGGGACTACTTCCCCGAACCG

GTGACGGTGTCGTGGAACTCAGGCGCCCTGACCAGC

GGCGTGCACACCTTCCCGGCTGTCCTACAGTCCTCA

GGACTCTACTCCCTCAGCAGCGTGGTGACCGTGCCC

TCCAGCAGCTTGGGCACCCAGACCTACATCTGCAAC

GTGAATCACAAGCCCAGCAACACCAAGGTGGACAAG

AAAGTT


h-segment;

GAGCCCAAATCTTGTGACAAAACTCACACATGCCCA

CCGTGCCCA

# Fig. 7-2

$C_H2$-segment;

GCACCTGAACTCCTGGGGGGACCGTCAGTCTTCCTC

TTCCCCCCAAAACCCAAGGACACCCTCATGATCTCC

CGGACCCCTGAGGTCACATGCGTGGTGGTGGACGTG

AGCCACGAAGACCCTGAGGTCAAGTTCAACTGGTAC

GTGGACGGCGTGGAGGTGCATAATGCCAAGACAAAG

CCGCGGGAGGAGCAGTACAACAGCACGTACCGGGTG

GTCAGCGTCCTCACCGTCCTGCACCAGGACTGGCTG

AATGGCAAGGAGTACAAGTGCAAGGTCTCCAACAAA

GCCCTCCCAGCCCCCATCGAGAAAACCATCTCCAAA

GCCAAA


$C_H3$-segment;

GGGCAGCCCCGAGAACCACAGGTGTACACCCTGCCC

CCATCCCGGGAGGAGATGACCAAGAACCAGGTCAGC

CTGACCTGCCTGGTCAAAGGCTTCTATCCCAGCGAC

ATCGCCGTGGAGTGGGAGAGCAATGGGCAGCCGGAG

AACAACTACAAGACCACGCCTCCCGTGCTGGACTCC

GACGGCTCCTTCTTCCTCTATAGCAAGCTCACCGTG

GACAAGAGCAGGTGGCAGCAGGGGAACGTCTTCTCA

TGCTCCGTGATGCATGAGGCTCTGCACAACCACTAC

ACGCAGAAGAGCCTCTCCCTGTCCCCGGGTAAA

# Fig. 8 - 1

```
                                                          50
GTACTCAATT GAACATCCCT TGTTTAGTGT ATCAAATCAA GCTGGTCTTA

                                                         100
AGAGAAATCA ATGAACAATA CTTTGTGACA GATATGCACC ACAAAAGAGG

                                                         150
AAGAGAAAAA CAGAAATTTC CTTCTTTGTA AGTGTAGTGT CTACAAATAA

                                                         200
GTAGATATTG GTCCTAGATT AGCCAGGTTT GCCTTTGTTA ACAGACCACC

                                                         250
TGACTTTATA AGCCGAGAAC TCCAAAGACT ACTATTTGCA TAGTTCATCC

                                                         300
TCAGAAACCA CAAATTTCTC ACAGTTGTTT TAAAGAGATG CACTTATAGG

                                                         350
AAGAGCAATA ATTAGTCAGA GACCAGGATC AACAACACAA TGGATTTTCA

                                                         400
TGTGCAGATT TTCAGCTTCA TGCTAATCAG TGTCACAGGT AGAGATGGGT

                                                         450
AGGAGTTTGA GTTCAGAAAA CAAGGTTCTT TTTCCCAGGA AAGTTTCATA

                                                         500
AAGTAGACTT TTTCTTTCCT CTGAATATTG AATGCTATGA AATTATTTTT

                                                         550
GTATCTCTGT CTACTAACAC CCTCTATCTC TTTCACTCTC TCTTCTGTTG

                                                         600
TTTTTCCATA GTCATATTGT CCAGTGGAGA AATTGTGCTC ACCCAGTCTC

                                                         650
CAGCACTCAT GGCTGCTTCT CCAGGGGAGA AGGTCACCAT CCGCTGCAGT

                                                         700
GTCAGCTCAA GTATAAGTTC CAGCAACTTG CACTGGTACC AGCAGAAGTC
```

# Fig. 8-2

```
                                              750
AGAAACCTCC CCCAAACCCT GGATTTATGG CACATCCAAC CTGGCTTCTG

                                              800
GAGTCCCTGT TCGTTTCAGT GGCAGTGGAT CTGGGACCTC TTATTTTCTC

                                              850
ACAATCAGCA GCATGGAGGC TGAAGATGCT GCCACTTATT ACTGTCAACA

                                              900
GTGGAGTAGT TACCCACTCA CGTTCGGAGG GGGGACCAAG CTGGAAATAA

AACGT
```

# Fig. 9

ACTGTGGCTG CACCATCTGT CTTCATCTTC CCGCCATCTG ATGAGCAGTT GAAATCTGGA ACTGCCTCTG TTGTGTGCCT GCTGAATAAC TTCTATCCCA GAGAGGCCAA AGTACAGTGG AAGGTGGATA ACGCCCTCCA ATCGGGTAAC TCCCAGGAGA GTGTCACAGA GCAGGACAGC AAGGACAGCA CCTACAGCCT CAGCAGCACC CTGACGCTGA GCAAAGCAGA CTACGAGAAA CACAAAGTCT ACGCCTGCGA AGTCACCCAT CAGGGCCTGA GCTCGCCCGT CACAAAGAGC

TTCAACAGGG GAGAGTGT

# Fig. 10

a    0.3 Kb
b    0.4 Kb
c    0.2 Kb
d    0.01 Kb
e    0.02 Kb

0 255 694

## Fig. 11

(B-1)  EcoRI  AccI  SacI  (B-2) EcoRI  AccI

SacI  SacI

$C_\kappa$

0.6 Kbp   1.3 Kbp   0.7 Kbp

0.5 Kbp

2.6 Kbp

## Fig. 12

EcoRI

EcoRI
7.9 (Kbp)

0.3   1.4   2.0   2.5   3.0  3.3  3.7   4.7   6.5

$J_i$-D-V

PvuII   HindIII  BamHI  SphI  PvuII  SphI  SphI   EcoRV   EcoRV

0 255 694

Fig. 13-1

( TO Fig. 13-2 )

# Fig. 13-2

( FROM Fig. 13-1 )

Fig. 14-1

0 255 694

# Fig. 14-2

# Fig.15

PROBE V~NL-1~

NL-1    J558L-H

−1.7Kb

PROBE C~r1~

ARH    J558L-H

−1.7Kb

# Fig. 16

NON-REDUCING
CONDITION

$-H_2L_2$

REDUCING
CONDITION

$-H$

$-L$

# Fig. 17

PROBE
$V_{NL-1}$

PROBE
$C_\kappa$

## Fig. 18

REDUCING
CONDITION

NON-REDUCING
CONDITION

160K

54K ——— H

28K ━━━ L

# Fig. 19

X63Ag8.653

X63Ag8653-HL

CELL NUMBERS

FLUORESCENCE INTENSITY

# Fig. 20

0 255 694

# Fig. 21

EcoRI-(1)　　HindⅢ-(1)　　HindⅢ-(2)　　　　　　　　　HindⅢ-(3)　EcoRI-(2)

XbaI　　　　　　　　　　　XhoI

about 3.4Kb ← about 3.6Kb ← about 8.2Kb → about 5.8Kb →

I

Ⅱ-A　　　　Ⅱ-B

Ⅱ-A-(1)　　Ⅱ-A-(2)

# Fig. 23

pSV2-HIGI

Fig. 22

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 184 187 (TEIJIN LTD)<br><br>* Whole document * | 13-17, 25-27, 32,33 | C 12 N 15/00<br>A 61 K 39/395<br>C 12 N 5/00<br>C 12 P 21/00 |
| A | | 1-12, 18-24, 31 | |
| | --- | | |
| X | PROCEEDINGS OF THE NATIONAL ACADEMY OF SCIENCES OF THE USA, vol. 79, July 1982, pages 4386-4390; R. UEDA et al.: "Serological analysis of cell surface antigens of null cell acute lymphocytic leukemia by mouse monoclonal antibodies"<br>* Whole article * | 28-30 | |
| | --- | | **TECHNICAL FIELDS SEARCHED (Int. Cl.4)** |
| X,P | CANCER RESEARCH, vol. 47, 15th February 1987, pages 999-1005; Y. NISHIMURA et al.: "Recombinant human-mouse chimeric monoclonal antibody specific for common acute lymphocytic leukemia antigen"<br>* Whole article * | 1-35 | C 12 N<br>C 12 P |
| | ----- | | |

The present search report has been drawn up for all claims

| Place of search | Date of completion of the search | Examiner |
|---|---|---|
| THE HAGUE | 29-09-1987 | CUPIDO M. |